# EUROPEAN PATENT SPECIFICATION

(11) **EP 4 312 962 B1**
(45) Date of publication and mention of the grant of the patent: **12.03.2025**
(21) Application number: 22729562.3
(22) Date of filing: 17.05.2022
(51) Int. Cl.: A61K 8/49, A61Q 13/00, C07D 313/00, C11B 9/00, A61Q 5/02, A61Q 19/10, C11D 3/50

(54) **POWDERY, MUSKY ODORANT MACROCYCLES AND THEIR USE IN PERFUME COMPOSITIONS**
PULVERIGE, MUSKIGE GERUCHSSTOFF-MAKROZYKLEN UND IHRE VERWENDUNG IN PARFÜMKOMPOSITIONEN
MACROCYCLES ODORANTS POUDREUX, MUSQUÉS ET LEUR UTILISATION DANS DES COMPOSITIONS PARFUMANTES

(30) Priority: 20.05.2021 EP 21175067
(43) Date of publication of application: 07.02.2024
(73) Proprietor: Firmenich SA, 1242 Satigny (CH)
(72) Inventor: BIRKBECK, Anthony, Alexander, 1242 Satigny (CH); MADDALENA, Umberto, 1242 Satigny (CH)
(74) Representative: Strych, Sebastian
(86) International application number: PCT/EP2022/063301
(87) International publication number: WO 2022/243300

(56) References cited:
- WO-A1-2021/122945
- DE-T2- 69 028 755
- US-A1- 2009 306 411
- US-A1- 2017 211 014
- LEHMANN J ET AL: "Synthesis and Olfactory Properties of Regioisomeric Alkynolides and (Z)-Alkenolides", TETRAHEDRON, ELSEVIER SIENCE PUBLISHERS, AMSTERDAM, NL, vol. 55, no. 9, 26 February 1999 (1999-02-26), pages 2639 - 2658, XP004156778, ISSN: 0040-4020, DOI: 10.1016/S0040-4020(99)00041-1
- MASAYOSHI ANDO ET AL: "A MILD AND STEREOSPECIFIC CONVERSION OF VICINAL DIOLS INTO OLEFINS VIA 2-METHOXY-1, 3-DIOXOLANE DERIVATIVES", CHEMISTRY LETTERS, 1 January 1986 (1986-01-01), pages 879 - 882, XP055706083, Retrieved from the Internet <URL:https://www.journal.csj.jp/doi/pdf/10.1246/cl.1986.879>

## Description

### Technical field

The present invention relates to the field of perfumery. More particularly, it concerns a composition of matter comprising
a) 0.5 to 98.8% w/w of (*Z*)-oxacyclohexadec-12-en-2-one;
b) 0.1 to 10% w/w of (*E*)-oxacyclohexadec-12-en-2-one;
c) 0 to 10% w/w of (*E*)-oxacyclohexadec-13-en-2-one; and
d) 0.5 to 98.8% w/w of (*Z*)-oxacyclohexadec-13-en-2-one.

the percentage being relative to the total weight of the composition of matter; and
wherein the weight ratio of the *E*-diastereoisomers to the *Z*-diastereoisomers is comprised in the range between 14:86 and 0.5:99.5.

Said composition of matter is a useful perfumery ingredient, and therefore the present invention comprises the invention composition of matter as part of a perfuming composition or of a perfumed consumer product.

### Background of the invention

Ingredients imparting musk notes are very appreciated and widely used in perfumery as they are considered as one of a key perfumery base notes, in particular macrocyclic musk compounds. Several of said macrocyclic musk compounds possess a double bond leading in general to a mixture of diastereoisomers and / or regioisomers. One example is Habanolide^{®} (Firmenich SA, Geneva, Switzerland) comprising mainly: (*Z*)-oxacyclohexadec-12-en-2-one, (*E*)-oxacyclohexadec-12-en-2-one, (*E*)-oxacyclohexadec-13-en-2-one and (*Z*)-oxacyclohexadec-13-en-2-one with a *E* : *Z* ratio comprised between 70:30 and 80:20. The isomeric distribution has an impact on the organoleptic properties of the ingredient. So there is a need to develop routes to novel qualities in order to enrich the perfumer's palette. In particular, there is a need to reinforce the nitro-musk aspect of Habanolide^{®} while maintaining the ambrette facet. However, the exact organoleptic properties of each of the individual isomers are unknown and the actual syntheses leading to pure isomers or to mixtures comprise of mainly the *E* isomers.

For example, in WO2018104856, a method is reported to obtain (*E*)-oxacyclohexadec-12-en-2-one or (*E*)-oxacyclohexadec-13-en-2-one in high purity. Additionally, US2017211014 discloses the isomerization of Globalide^{®} leading to a mixture of oxacyclohexadec-12-en-2-one and oxacyclohexadec-13-en-2-one with a *E:Z* ratio of 65:31.

DE69028755T discloses a mixture of mainly E-oxacyclohexadec-12-en-2-one and Z-oxacyclohexadec-13-en-2-one, with the Z-isomers being present only in a small percentage.

US2009/306411 refers to a process for preparing saturated or unsaturated lactones cis-15-pentadec-11-enolide, trans-15-pentadec-11-enolide.

WO2021/122945 discloses compositions comprising a) 0.5 to 85% w/w of (Z)-oxacyclohexadec-12-en-2-one; b) 0.5 to 30% w/w of (E)-oxacyclohexadec-12-en-2-one; c) 0 to 30% w/w of (E)-oxacyclohexadec-13-en-2-one; and d) 0.5 to 85% w/w of (Z)-oxacyclohexadec-13-en-2-one, wherein the weight ratio of the E-diastereoisomers to the Z-diastereoisomers is comprised in the range between 40:60 and 15:85.

The present invention provides a novel composition of matter comprising a high ratio of *Z* to *E* isomers leading to a strong powdery/musk note very appreciated in perfumery. The prior art does not anticipate that the present composition of matter provides such an increase in performance, odour character and does not provide any method to significantly enrich the Z isomers.

### Description of the invention

A surprising synergic effect has been discovered between various isomers of oxacyclohexadec-(12 or 13)-en-2-one leading to the invention composition of matter possessing a powerful musky/powdery note combined with an ambrette aspect.

So, a first object of the present invention is a composition of matter comprising:
a) 0.5 to 98.8% w/w of (*Z*)-oxacyclohexadec-12-en-2-one;
b) 0.1 to 10% w/w of (*E*)-oxacyclohexadec-12-en-2-one;
c) 0 to 10% w/w of (*E*)-oxacyclohexadec-13-en-2-one; and
d) 0.5 to 98.8% w/w of (*Z*)-oxacyclohexadec-13-en-2-one.

the percentage being relative to the total weight of the composition of matter; and
wherein the weight ratio of the *E*-diastereoisomers to the *Z*-diastereoisomers is comprised in the range between 14:86 and 0.5:99.5.

Said composition of matter can be used as perfuming ingredient, for instance to impart odor notes of the powdery, musk type and also having an amber aspect.

By the terms "*E*-diastereoisomers" and "*Z*-diastereoisomers", it is meant the normal meaning understood by a person skilled in the art, i.e the *E*-diastereoisomers correspond to (E)-oxacyclohexadec-12-en-2-one and (*E*)-oxacyclohexadec-13-en-2-one and *Z-*diastereoisomers correspond to (*Z*)-oxacyclohexadec-12-en-2-one and (*Z*)-oxacyclohexadec-13-en-2-one.

According to an embodiment of the invention, in the present composition of matter the various constituents mentioned above are present in the following amounts:
a) 55 to 98.8% w/w of (Z)-oxacyclohexadec-12-en-2-one;
b) 0.1 to 7% w/w of (E)-oxacyclohexadec-12-en-2-one;
c) 0 to 6% w/w of (E)-oxacyclohexadec-13-en-2-one; and
d) 0.5 to 31% w/w of (Z)-oxacyclohexadec-13-en-2-one.

According to an embodiment of the invention, in the present composition of matter the various constituents mentioned above are present in the following amounts:
a) 90 to 98.8% w/w of (Z)-oxacyclohexadec-12-en-2-one;
b) 0.1 to 2.5% w/w of (E)-oxacyclohexadec-12-en-2-one;
c) 0 to 1% w/w of (E)-oxacyclohexadec-13-en-2-one; and
d) 0.5 to 2.5% w/w of (Z)-oxacyclohexadec-13-en-2-one.

According to an embodiment of the invention, in the present composition of matter the various constituents mentioned above are present in the following amounts:
a) 0.5 to 10% w/w of (*Z*)-oxacyclohexadec-12-en-2-one;
b) 0.1 to 2.5% w/w of (*E*)-oxacyclohexadec-12-en-2-one;
c) 0 to 3% w/w of (*E*)-oxacyclohexadec-13-en-2-one; and
d) 55 to 98.8% w/w of (*Z*)-oxacyclohexadec-13-en-2-one.

According to any one of the above embodiments of the invention, the present composition of matter may comprise from about 58 to 98.8% w/w of (Z)-oxacyclohexadec-12-en-2-one, particularly from about 58 to 70% w/w or from about 92 to 98.8% w/w of (Z)-oxacyclohexadec-12-en-2-one, more particularly from about 58 to 65% w/w or from about 95 to 98.8% w/w of (Z)-oxacyclohexadec-12-en-2-one.

According to a particular embodiment of the invention, the present composition of matter may comprise from about 0.5 to 15% w/w of (Z)-oxacyclohexadec-12-en-2-one, particularly from 1 to 10% w/w of (Z)-oxacyclohexadec-12-en-2-one, particularly from 1.5 to 8% w/w of (Z)-oxacyclohexadec-12-en-2-one, even more particularly from 3 to 7% w/w of (Z)-oxacyclohexadec-12-en-2-one.

According to any one of the above embodiments of the invention, the present composition of matter may comprise from about 0.1 to 10% w/w of (*E*)-oxacyclohexadec-12-en-2-one, particularly, from about 0. 1 to 8% w/w of (*E*)-oxacyclohexadec-12-en-2-one, particularly from about 0.1 to 7% w/w of (*E*)-oxacyclohexadec-12-en-2-one, particularly, from about 0.1 to 6% w/w of (*E*)-oxacyclohexadec-12-en-2-one, particularly from about 0.1 to 5% w/w of (*E*)-oxacyclohexadec-12-en-2-one, particularly from about 0.1 to 2% w/w of (*E*)-oxacyclohexadec-12-en-2-one and more particularly from about 0.1 to 1.5% w/w of (*E*)-oxacyclohexadec-12-en-2-one.

According to any one of the above embodiments of the invention, the present composition of matter may comprise from about 1 to 97% w/w of (Z)-oxacyclohexadec-13-en-2-one, particularly from about 1.5 to 96% w/w of (Z)-oxacyclohexadec-13-en-2-one, particularly from about 2 to 96% w/w of (Z)-oxacyclohexadec-13-en-2-one, particularly from about 2.5 to 96% w/w of (Z)-oxacyclohexadec-13-en-2-one, particularly from about 2.5 to 95% w/w of (Z)-oxacyclohexadec-13-en-2-one, particularly from about 10 to 95% w/w of (Z)-oxacyclohexadec-13-en-2-one, particularly from about 20 to 95% w/w of (Z)-oxacyclohexadec-13-en-2-one, particularly from about 90 to 95% w/w or from about 1.5 to 31% w/w of (Z)-oxacyclohexadec-13-en-2-one, even more particularly from about 90 to 95% w/w or from about 5 to 31% w/w of (Z)-oxacyclohexadec-13-en-2-one.

According to any one of the above embodiments of the invention, the present composition of matter may comprise from about 0 to 10% w/w of (E)-oxacyclohexadec-13-en-2-one, particularly from about 0 to 7% w/w of (E)-oxacyclohexadec-13-en-2-one and more particularly from about 0 to 5% w/w of (*E*)-oxacyclohexadec-13-en-2-one.

According to any one of the above embodiments of the invention, the weight ratio of the *E*-diastereoisomers to the *Z*-diastereoisomers is comprised in the range between 12:88 and 1:99, particularly in the range between 10:90 and 1:99, particularly in the range between 9.5:90.5 and 1:99, particularly in the range between 7.5:92.5 and 1:99, particularly in the range between 5:95 and 2:98; and more particularly in the range between 3:97 and 2:98.

According to any one of the above embodiments of the invention, the weight ratio of the 12-regioisomers to the 13-regioisomers is comprised in the range between 55:45 and 99:1 or in the range between 20:80 and 1:99; more particularly in the range between 65:35 and 99:1 or in the range between 10:90 and 5:95.

By the terms "12-regioisomers" and "13-regioisomers", it is meant the normal meaning understood by a person skilled in the art, i.e the 12-regioisomers correspond to (E)-oxacyclohexadec-12-en-2-one and (*Z*)-oxacyclohexadec-12-en-2-one and 13-regioisomers correspond to (*E*)-oxacyclohexadec-13-en-2-one and (Z)-oxacyclohexadec-13-en-2-one.

As mentioned above, the composition of matter of the invention possesses a very powerful musk and tenacious odor with a stronger than expected powdery note in the direction of nitro musks. The overall odour profile is highly appreciated by perfumers since it opens up new directions in the perfumer's creativity when compared with the prior art ingredient Habanolide^{®}.

Indeed, when the odor of the invention's composition of matter is compared with that of the prior art Habanolide^{®}, then the invention's compositions of matter distinguish themselves by a clearly different odor profile characterized by a stronger powdery and ambrette note and by much weaker metallic note, so characteristic of the prior art compound. Moreover, the invention's compositions of matter impart a clearly stronger and more substantive note while bringing more volume to perfuming composition. The invention's compositions of matter distinguish themselves also by showing a more appreciated olfactive profile. Overall, while the Habanolide^{®} is more in the macrocyclic musk direction with a slight metallic aspect, the present composition of matter is more on the nitro musk direction which is particularly sought after due to the highly restricted use of nitro musks.

Said differences lend the invention's compositions of matter and the prior art compounds to each be suitable for different uses, i.e. to impart different organoleptic impressions.

As mentioned above, the invention concerns the use of the invention's compositions of matter as a perfuming ingredient. In other words, it concerns a method or a process to confer, enhance, improve or modify the odor properties of a perfuming composition or of a perfumed article or of a surface, which method comprises adding to said composition or article an effective amount the invention's composition of matter, e.g. to impart its typical note. Understood that the final hedonic effect may depend on the precise dosage and on the organoleptic properties of the invention's composition of matter, but anyway the addition of the invention's compositions of matter will impart to the final product its typical touch in the form of a note, touch or aspect depending on the dosage. In addition, the invention's composition of matter may also be used to enhance the organoleptic properties of perfuming ingredient or to decrease the unpleasant olfactory impression such as metallic, dusty or chemical facet of some perfuming ingredients.

By "use of the invention's compositions of matter it has to be understood here also the use of any composition containing the invention's compositions of matter and which can be advantageously employed in the perfumery industry.

Said compositions, which in fact can be advantageously employed as perfuming ingredients, are also an object of the present invention.

Therefore, another object of the present invention is a perfuming composition comprising:
i) as a perfuming ingredient, at least one invention's composition of matter as defined above;
ii) at least one ingredient selected from the group consisting of a perfumery carrier and a perfumery base; and
iii) optionally at least one perfumery adjuvant.

By "perfumery carrier" it is meant here a material which is practically neutral from a perfumery point of view, i.e. that does not significantly alter the organoleptic properties of perfuming ingredients. Said carrier may be a liquid or a solid.

As liquid carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples, solvents such as butylene or propylene glycol, glycerol, dipropyleneglycol and its monoether, 1,2,3-propanetriyl triacetate, dimethyl glutarate, dimethyl adipate 1,3-diacetyloxypropan-2-yl acetate, diethyl phthalate, isopropyl myristate, Abalyn^{®} (rosin resins, available from Eastman), benzyl benzoate, benzyl alcohol, 2-(2-ethoxyethoxy)-1-ethanol, tri-ethyl citrate or mixtures thereof, which are the most commonly used or also naturally derived solvents like glycerol or various vegetable oils such as palm oil, sunflower oil or linseed oil. For the compositions which comprise both a perfumery carrier and a perfumery base, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company), or hydrogenated castors oils such as those known under the trademark Cremophor^{®} RH 40 (origin: BASF).

Solid carrier is meant to designate a material to which the perfuming composition or some element of the perfuming composition can be chemically or physically bound. In general such solid carriers are employed either to stabilize the composition, or to control the rate of evaporation of the compositions or of some ingredients. Solid carriers are of current use in the art and a person skilled in the art knows how to reach the desired effect. However by way of non-limiting examples of solid carriers, one may cite absorbing gums or polymers or inorganic materials, such as porous polymers, cyclodextrin, dextrin, maltodextrin wood based materials, organic or inorganic gels, clays, gypsum talc or zeolites.

As other non-limiting examples of solid carriers, one may cite encapsulating materials. Examples of such materials may comprise wall-forming and plasticizing materials, such as glucose syrups, natural or modified starches, hydrocolloids, cellulose derivatives, polyvinyl acetates, polyvinylalcohols, proteins or pectins, plant gums such as acacia gum (Gum Arabic), urea, sodium chloride, sodium sulphate, zeolite, sodium carbonate, sodium bicarbonate, clay, talc, calcium carbonate, magnesium sulfate, gypsum, calcium sulfate, magnesium oxide, zinc oxide, titanium dioxide, calcium chloride, potassium chloride, magnesium chloride, zinc chloride, carbohydrates, saccharides such as sucrose, mono-, di-, tri- and polysaccharides and derivatives such as chitosan, starch, cellulose, carboxymethyl methylcellulose, methylcellulose, hydroxyethyl cellulose, ethyl cellulose, propyl cellulose, polyols/sugar alcohols such as sorbitol, maltitol, xylitol, erythritol, and isomalt, polyethylene glycol (PEG), polyvinyl pyrrolidin (PVP), polyvinyl alcohol, acrylamides, acrylates, polyacrylic acid and related, maleic anhydride copolymers, amine-functional polymers, vinyl ethers, styrenes, polystyrenesulfonates, vinyl acids, ethylene glycol-propylene glycol block copolymers, vegetable gums, gum acacia, pectins, xanthanes, alginates, carragenans, citric acid or any water soluble solid acid, fatty alcohols or fatty acids and mixtures thereof, or yet the materials cited in reference texts such as H. Scherz, Hydrokolloide: Stabilisatoren, Dickungs- und Geliermittel in Lebensmitteln, Band 2 der Schriftenreihe Lebensmittelchemie, Lebensmittelqualität, Behr's Verlag GmbH & Co., Hamburg, 1996. The encapsulation is a well-known process to a person skilled in the art, and may be performed, for instance, by using techniques such as spray-drying, agglomeration or yet extrusion; or consists of a coating encapsulation, including coacervation and complex coacervation techniques.

As non-limiting examples of solid carriers, one may cite in particular the core-shell capsules with resins of aminoplast, polyamide, polyester, polyurea or polyurethane type or a mixture thereof (all of said resins are well known to a person skilled in the art) using techniques like phase separation process induced by polymerization, interfacial polymerization, coacervation or altogether (all of said techniques have been described in the prior art), optionally in the presence of a polymeric stabilizer or of a cationic copolymer.

Resins may be produced by the polycondensation of an aldehyde (e.g. formaldehyde, 2,2-dimethoxyethanal, glyoxal, glyoxylic acid or glycolaldehyde and mixtures thereof) with an amine such as urea, benzoguanamine, glycouril, melamine, methylol melamine, methylated methylol melamine, guanazole and the like, as well as mixtures thereof. Alternatively one may use preformed resins alkylolated polyamines such as those commercially available under the trademark Urac^{®} (origin: Cytec Technology Corp.), Cymel^{®} (origin: Cytec Technology Corp.), Urecoll^{®} or Luracoll^{®} (origin: BASF).

Other resins are the ones produced by the polycondensation of a polyol, like glycerol, and a polyisocyanate, like a trimer of hexamethylene diisocyanate, a trimer of isophorone diisocyanate or xylylene diisocyanate or a Biuret of hexamethylene diisocyanate or a trimer of xylylene diisocyanate with trimethylolpropane (known with the tradename of Takenate^{®}, origin: Mitsui Chemicals), among which a trimer of xylylene diisocyanate with trimethylolpropane and a Biuret of hexamethylene diisocyanate are preferred.

Some of the seminal literature related to the encapsulation of perfumes by polycondensation of amino resins, namely melamine based resins with aldehydes includes articles such as those published by K. Dietrich et al. Acta Polymerica, 1989, vol. 40, pages 243, 325 and 683, as well as 1990, vol. 41, page 91. Such articles already describe the various parameters affecting the preparation of such core-shell microcapsules following prior art methods that are also further detailed and exemplified in the patent literature. US 4'396'670, to the Wiggins Teape Group Limited is a pertinent early example of the latter. Since then, many other authors have enriched the literature in this field and it would be impossible to cover all published developments here, but the general knowledge in encapsulation technology is very significant. More recent publications of pertinence, which disclose suitable uses of such microcapsules, are represented for example by the article of K. Bruyninckx and M. Dusselier, ACS Sustainable Chemistry & Engineering, 2019, vol. 7, pages 8041-8054.

By "perfumery base" what is meant here is a composition comprising at least one perfuming co-ingredient.

By "perfuming co-ingredient" it is meant here a compound, which is used in a perfuming preparation or a composition to impart a hedonic effect; i.e. used for the primary purpose of conferring or modulating an odor. In other words such a co-ingredient, to be considered as being a perfuming one, must be recognized by a person skilled in the art as being able to impart or modify in a positive or pleasant way the odor of a composition, and not just as having an odor.

The nature and type of the perfuming co-ingredients present in the base do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin.

In particular, one may cite perfuming co-ingredients which are commonly used in perfume formulations, such as:
- Aldehydic ingredients: decanal, dodecanal, 2-methyl-undecanal, 10-undecenal, octanal, nonanal and/or nonenal;
- Aromatic-herbal ingredients: eucalyptus oil, camphor, eucalyptol, 5-methyltricyclo[6.2.1.0-2,7-]undecan-4-one, 1-methoxy-3-hexanethiol, 2-ethyl-4,4-dimethyl-1,3-oxathiane, 2,2,7/8,9/10-Tetramethylspiro[5.5]undec-8-en-1-one, menthol and/or alpha-pinene;
- Balsamic ingredients: coumarin, ethylvanillin and/or vanillin;
- Citrus ingredients: dihydromyrcenol, citral, orange oil, linalyl acetate, citronellyl nitrile, orange terpenes, limonene, 1-p-menthen-8-yl acetate and/or 1,4(8)-p-menthadiene;
- Floral ingredients:methyl dihydrojasmonate, linalool, citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, (1E)-1-(2,6,6-trimethyl-2-cyclohexen-1-yl)-1-penten-3-one, 1-(2,6,6-trimethyl-1,3-cyclohexadien-1-yl)-2-buten-1-one, (2E)-1-(2,6,6-trimethyl-2-cyclohexen-1-yl)-2-buten-1-one, (2E)-1-[2,6,6-trimethyl-3-cyclohexen-1-yl]-2-buten-1-one, (2E)-1-(2,6,6-trimethyl-1-cyclohexen-1-yl)-2-buten-1-one, 2,5-dimethyl-2-indanmethanol, 2,6,6-trimethyl-3-cyclohexene-1-carboxylate, 3-(4,4-dimethyl-1-cyclohexen-1-yl)propanal, hexyl salicylate, 3,7-dimethyl-1,6-nonadien-3-ol, 3-(4-isopropylphenyl)-2-methylpropanal, verdyl acetate, geraniol, p-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate , high cis methyl dihydrojasmonate, 3-methyl-5-phenyl-1-pentanol, verdyl proprionate, geranyl acetate, tetrahydro linalool, cis-7-p-menthanol, propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 8-decen-5-olide, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma-undecalactone, 2,2,5-trimethyl-5-pentylcyclopentanone, 2-methyl-4-propyl-1,3-oxathiane, 4-decanolide, ethyl 2-methyl-pentanoate, hexyl acetate, ethyl 2-methylbutanoate, gamma-nonalactone, allyl heptanoate, 2-phenoxyethyl isobutyrate, ethyl 2-methyl-1,3-dioxolane-2-acetate, 3-(3,3/1,1-dimethyl-5-indanyl)propanal, diethyl 1,4-cyclohexanedicarboxylate, 3-methyl-2-hexen-1-yl acetate, 1-[3,3-dimethylcyclohexyl]ethyl [3-ethyl-2-oxiranyl]acetate and/or diethyl 1,4-cyclohexane dicarboxylate;
- Green ingredients: 2-methyl-3-hexanone (E)-oxime, 2,4-dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, (Z)-4-cyclopentadecen-1-one, 3-methylcyclopentadecanone, 1-oxa-12-cyclohexadecen-2-one, 1-oxa-13-cyclohexadecen-2-one, (9Z)-9-cycloheptadecen-1-one, 2-{(1S)-1-[(1R)-3,3-dimethylcyclohexyl]ethoxy}-2-oxoethyl propionate 3-methyl-5-cyclopentadecen-1-one, 1,3,4,6,7,8-hexahydro-4,6,6,7,8,8-hexamethyl-cyclopenta-g-2-benzopyrane, (1S,1'R)-2-[1-(3',3'-dimethyl-1'-cyclohexyl)ethoxy]-2-methylpropyl propanoate, oxacyclohexadecan-2-oneand/or (1S,1'R)-[1-(3',3'-dimethyl-1'-cyclohexyl)ethoxycarbonyl]methyl propanoate, ;
- Woody ingredients: 1-[(1RS,6SR)-2,2,6-trimethylcyclohexyl]-3-hexanol, 3,3-dimethyl-5-[(1R)-2,2,3-trimethyl-3-cyclopenten-1-yl]-4-penten-2-ol, 3,4'-dimethylspiro[oxirane-2,9'-tricyclo[6.2. 1.0^{2,7}]undec[4]ene, (1-ethoxyethoxy)cyclododecane, 2,2,9,11-tetramethylspiro[5.5]undec-8-en-1-yl acetate, 1-(octahydro-2,3,8,8-tetramethyl-2-naphtalenyl)-1-ethanone, patchouli oil, terpenes fractions of patchouli oil, Clearwood^{®} (Origin: Firmenich SA), (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal, 7-methyl-2H-1,5-benzodioxepin-3(4H)-one, 2,5,5-trimethyl-1,2,3,4,4a,5,6,7-octahydro-2-naphthalenol, 1-phenylvinyl acetate, 6-methyl-7-oxa-1-thia-4-azaspiro[4.4]nonan and/or 3-(3-isopropyl-1-phenyl)butanal.

According to a particular embodiment, the invention's perfuming composition comprises, as a perfuming co-ingredient, at least one woody ingredient.

A perfumery base according to the invention may not be limited to the above mentioned perfuming co-ingredients, and many other of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds also known as properfume or profragrance. Non-limiting examples of suitable properfumes may include 4-(dodecylthio)-4-(2,6,6-trimethyl-2-cyclohexen-1-yl)-2-butanone, 4-(dodecylthio)-4-(2,6,6-trimethyl-1-cyclohexen-1-yl)-2-butanone, trans-3-(dodecylthio)-1-(2,6,6-trimethyl-3-cyclohexen-1-yl)-1-butanone, 3-(dodecylsulfonyl)-1-(2,6,6-trimethylcyclohex-3-en-1-yl)butan-1-one, a linear polysiloxane co-polymer of (3-mercaptopropyl)(methyl)dimethoxysilane, 2-(dodecylthio)octan-4-one, 2-(dodecylsulfonyl)octan-4-one, 4-oxooctan-2-yl dodecanoate, 2-phenylethyl oxo(phenyl)acetate, 3,7-dimethylocta-2,6-dien-1-yl oxo(phenyl)acetate, (Z)-hex-3-en-1-yl oxo(phenyl)acetate, 3,7-dimethyl-2,6-octadien-1-yl hexadecanoate, bis(3,7-dimethylocta-2,6-dien-1-yl) succinate, (2E,6Z)-2,6-nonadienyl hexadecanoate, (2E,6Z)-2,6-nonadien-1-yl tetradecanoate, (2E,6Z)-2,6-nonadien-1-yl dodecanoate, (2-((2-methylundec-1-en-1-yl)oxy)ethyl)benzene, 1-methoxy-4-(3-methyl-4-phenethoxybut-3-en-1-yl)benzene, (3-methyl-4-phenethoxybut-3-en-1-yl)benzene, 1-(((Z)-hex-3-en-1-yl)oxy)-2-methylundec-1-ene, (2-((2-methylundec-1-en-1-yl)oxy)ethoxy)benzene, 2-methyl-1-(octan-3-yloxy)undec-1-ene, 1-methoxy-4-(1-phenethoxyprop-1-en-2-yl)benzene, 1-methyl-4-(1-phenethoxyprop-1-en-2-yl)benzene, 2-(1-phenethoxyprop-1-en-2-yl)naphthalene, (2-phenethoxyvinyl)benzene, 2-(1-((3,7-dimethyloct-6-en-1-yl)oxy)prop-1-en-2-yl)naphthalene, (2-((2-pentylcyclopentylidene)methoxy)ethyl)benzene, 4-allyl-2-methoxy-1-((2-methoxy-2-phenylvinyl)oxy)benzene, (2-((2-heptylcyclopentylidene)methoxy)ethyl)benzene, 1-methoxy-4-(1-phenethoxyprop-1-en-2-yl)benzene, (2-((2-methyl-4-(2,6,6-trimethylcyclohex-1-en-1-yl)but-1-en-1-yl)oxy)ethyl)benzene, 1-methoxy-4-(2-methyl-3-phenethoxyallyl)benzene, (2-((2-isopropyl-5-methylcyclohexylidene)methoxy)ethyl)benzene, 1-isopropyl-4-methyl-2-((2-pentylcyclopentylidene)methoxy)benzene, 2-methoxy-1-((2-pentylcyclopentylidene)methoxy)-4-propylbenzene, 2-ethoxy-1-((2-methoxy-2-phenylvinyl)oxy)-4-methylbenzene, 3-methoxy-4-((2-methoxy-2-phenylvinyl)oxy)benzaldehyde, 1-isopropyl-2-((2-methoxy-2-phenylvinyl)oxy)-4-methylbenzene, 4-((2-(hexyloxy)-2-phenylvinyl)oxy)-3-methoxybenzaldehyde or a mixture thereof.

By "perfumery adjuvant", it is meant here an ingredient capable of imparting additional added benefit such as a color, a particular light resistance, chemical stability, etc. A detailed description of the nature and type of adjuvant commonly used in perfuming composition cannot be exhaustive, but it has to be mentioned that said ingredients are well known to a person skilled in the art. One may cite as specific non-limiting examples the following: viscosity agents (e.g. surfactants, thickeners, gelling and/or rheology modifiers), stabilizing agents (e.g. preservatives, antioxidant, heat/light and or buffers or chelating agents, such as BHT), coloring agents (e.g. dyes and/or pigments), preservatives (e.g. antibacterial or antimicrobial or antifungal or anti irritant agents), abrasives, skin cooling agents, fixatives, insect repellants, ointments, vitamins and mixtures thereof. By "fixative" also called "modulator", it is understood here an agent having the capacity to affect the manner in which the odour, and in particular the evaporation rate and intensity, of the compositions incorporating said modulator can be perceived by an observer or user thereof, over time, as compared to the same perception in the absence of the modulator. In particular, the modulator allows prolonging the time during which their fragrance is perceived. Non-limiting examples of suitable modulators may include methyl glucoside polyol; ethyl glucoside polyol; propyl glucoside polyol; isocetyl alcohol; PPG-3 myristyl ether; neopentyl glycol diethylhexanoate; sucrose laurate; sucrose dilaurate, sucrose myristate, sucrose palmitate, sucrose stearate, sucrose distearate, sucrose tristearate, hyaluronic acid disaccharide sodium salt, sodium hyaluronate, propylene glycol propyl ether; dicetyl ether; polyglycerin-4 ethers; isoceteth-5; isoceteth-7, isoceteth-10; isoceteth-12; isoceteth-15; isoceteth-20; isoceteth-25; isoceteth-30; disodium lauroamphodipropionate; hexaethylene glycol monododecyl ether; and their mixtures; neopentyl glycol diisononanoate; cetearyl ethylhexanoate; panthenol ethyl ether, DL-panthenol, N-hexadecyl n-nonanoate, noctadecyl n-nonanoate, a profragrance, cyclodextrin, an encapsulation, and a combination thereof. At most 20% by weight, based on the total weight of the perfuming composition, of the modulator may be incorporated into the perfumed consumer product.

It is understood that a person skilled in the art is perfectly able to design optimal formulations for the desired effect by admixing the above mentioned components of a perfuming composition, simply by applying the standard knowledge of the art as well as by trial and error methodologies.

An invention's composition consisting of at least one composition of matter as defined above and at least one perfumery carrier consists of a particular embodiment of the invention as well as a perfuming composition comprising at least one composition of matter as defined above, at least one perfumery carrier, at least one perfumery base, and optionally at least one perfumery adjuvant.

For the sake of clarity, it is also understood that any mixture resulting directly from a chemical synthesis, e.g. a reaction medium without an adequate purification, in which the composition of matter of the invention would be involved as a starting, intermediate or end-product could not be considered as a perfuming composition according to the invention as far as said mixture does not provide the inventive composition of matter in a suitable form for perfumery. Thus, unpurified reaction mixtures are generally excluded from the present invention unless otherwise specified.

The invention's composition of matter can also be advantageously used in all the fields of modern perfumery, i.e. fine or functional perfumery, to positively impart or modify the odor of a consumer product into which said composition of matter is added. Consequently, another object of the present invention consists of by a perfumed consumer product comprising, as a perfuming ingredient, at least one composition of matter, as defined above.

The invention's composition of matter can be added as such or as part of an invention's perfuming composition.

For the sake of clarity, "perfumed consumer product" is meant to designate a consumer product which delivers at least a pleasant perfuming effect to the surface or space to which it is applied (e.g. skin, hair, textile, or home surface). In other words, a perfumed consumer product according to the invention is a perfumed consumer product which comprises a functional formulation, as well as optionally additional benefit agents, corresponding to the desired consumer product, and an olfactive effective amount of at least one invention's composition of matter. For the sake of clarity, said perfumed consumer product is a non-edible product.

The nature and type of the constituents of the perfumed consumer product do not warrant a more detailed description here, which in any case would not be exhaustive, the skilled person being able to select them on the basis of his general knowledge and according to the nature and the desired effect of said product.

Non-limiting examples of suitable perfumed consumer products include a perfume, such as a fine perfume, a splash or eau de parfum, a cologne or a shave or after-shave lotion; a fabric care product, such as a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product; a body-care product, such as a hair care product (e.g. a shampoo, a leave-on or rinse-off hair conditioner, a coloring preparation or a hair spray, a color-care product, a hair shaping product, a dental care product), a disinfectant, an intimate care product; a cosmetic preparation (e.g. a skin cream or lotion, a vanishing cream or a deodorant or antiperspirant (e.g. a spray or roll on), a hair remover, a tanning or sun or after sun product, a nail product, a skin cleansing, a makeup); or a skin-care product (e.g. a soap, a shower or bath mousse, oil or gel, or a hygiene product or a foot/hand care products); an air care product, such as an air freshener or a "ready to use" powdered air freshener which can be used in the home space (rooms, refrigerators, cupboards, shoes or car) and/or in a public space (halls, hotels, malls, etc..); or a home care product, such as a mold remover, a furnisher care product, a wipe, a dish detergent or a hard-surface (e.g. a floor, bath, sanitary or a window-cleaning) detergent; a leather care product; a car care product, such as a polish, a wax or a plastic cleaner.

According to any embodiments of the invention, the perfumed consumer product of the invention are characterized by a pH of 1 or more. Particularly, the perfumed consumer product of the invention has a pH comprised between 1 and 12, or between 1 and 8. Even more particularly, the perfumed consumer product of the invention has a pH comprised between 1 and 6.

According to a particular embodiment, the invention's perfumed consumer product is in the form of a personal care, a home care or fabric care consumer product comprising ingredients that are common in the personal, home or fabric care consumer products, in particular shower gel, shampoo, soap, fabric detergents or softeners and all-purpose cleaners. The main functional constituents of perfumed consumer products are surfactants and/or softener components capable of cleaning and/or softening fabrics and/or textiles of varied nature, such as clothes, curtain fabrics, carpet and furniture fabrics, etc, or other home surfaces, skin or hair, and typically used in a large amount of water or water-based solvents. These are therefore formulations wherein the amount of water is typically comprised between 50 and 99% by weight of the perfumed consumer product with the exception of soap or solid detergent wherein the amount of water is at most 20%.

A more detailed description of such fabric cleaning and/or softening formulations is not warranted here, many descriptions of current liquid formulations can be found in the cleaner/fabric softener's patent and other pertinent literature, such as for example the textbook of Louis Ho Tan Tai, "Détergents et Produits de Soins Corporels, Chapters 1 to 7 in particular, Dunod, Paris, 1999, or any other similar and/or more recent textbooks pertaining to the art of liquid softener and all-purpose cleaners formulations. A patent publication, WO 2010/105873, is also cited by way of example, in as much as it describes typical current ingredients, other than perfumes, of such liquid products, particularly in pages 9 to 21. Of course, many other examples of liquid cleaner and/or fabric softener formulations can be found in the literature. Any such liquid formulations, namely liquid fabric cleaner or conditioner and/or all-purpose cleaner, can be used in the here-described compositions.

According to a particular embodiment of the invention, the invention's perfumed consumer product is a liquid fabric softener comprising a fabric softener active base in amount comprised between 85 and 100% by weight, based on the total weight of the perfumed consumer product. The main constituent of the fabric softener active base is water or water-based solvents. The fabric softener active base may comprise dialkyl quaternary ammonium salts, dialkyl ester quaternary ammonium salts, Hamburg esterquat, triethanolamine quat, silicones and mixtures thereof. Optionally, the fabric softener active base of the composition may further comprise a viscosity modifier in an amount comprised between 0.05 and 1% by weight, based on the total weight of the liquid base; preferably chosen in the group consisting of calcium chloride.

According to a particular embodiment of the invention, the invention's perfumed consumer product is an all-purpose cleaner comprising an all-purpose cleaner active base in amount comprised between 85 and 100% by weight, based on the total weight of the perfumed consumer product. The main constituent of the all-purpose cleaner active base is water or water-based solvents The all-purpose active base may comprise linear alkylbenzene sulfonates (LAS) in an amount comprised between 1 and 2%, nonionic surfactant in an amount comprised between 2 and 4% and acid such as citric acid in an amount comprised between 0.1 and 0.5%.

According to a particular embodiment of the invention, the invention's perfumed consumer product is a liquid detergent comprising a liquid detergent active base in amount comprised between 85 and 100% by weight, based on the total weight of the perfumed consumer product. The main constituent of the liquid detergent active base is water or water-based solvents. The liquid detergent active base may comprise anionic surfactant such as alkylbenzenesulfonate (ABS), linear alkylbenzene sulfonates (LAS), secondary alkyl sulfonate (SAS), primary alcohol sulfate (PAS), lauryl ether sulfate (LES), sodium lauryl ether sulfate (SLES), methyl ester sulfonate (MES); nonionic surfactant such as alkyl amines, alkanolamide, fatty alcohol poly(ethylene glycol) ether, fatty alcohol ethoxylate (FAE), ethylene oxide (EO) and propylene oxide (PO) copolymers, amine oxydes, alkyl polyglucosides, alkyl polyglucosamides; ormixtures thereof.

According to a particular embodiment of the invention, the invention's perfumed consumer product is a solid detergent comprising a solid detergent active base in amount comprised between 85 and 100% by weight, based on the total weight of the perfumed consumer product. The solid detergent active base may comprise at least one surfactant chosen in the group consisting of anionic, nonionic, cationic, zwiterionic surfactant and mixtures thereof. The surfactant in the solid detergent active base is preferably chosen in the group consisting of linear alkene benzene sulphonate (LABS), sodium laureth sulphate, sodium lauryl ether sulphate (SLES), sodium lauryl sulphate (SLS), alpha olefin sulphonate (AOS), methyl ester sulphonates (MES), alkyl polyglyucosides (APG), primary alcohol ethoxylates and in particular lauryl alcohol ethoxylates (LAE), primary alcohol sulphonates (PAS), soap and mixtures thereof. The soild detergent active base may comprise a further component, commonly used in powder detergent consumer product, selected from the group consisting of bleaching agents such as TAED (tetraacetylethylenediamine); buffering agent; builders such as zeolites, sodium carbonate or mixture thereof; soil release or soil suspension polymers; granulated enzyme particles such as cellulase, lipase, protease, mannanase, pectinase or mixtures thereof; corrosion inhibitor; antifoaming; sud suppressing agents; dyes; fillers such as sodium silicate, sodium sulfate or mixture thereof; source of hydrogen peroxide such as sodium percarbonate or sodium perborate; and mixtures thereof.

According to a particular embodiment of the invention, the invention's perfumed consumer product is shampoo or a shower gel comprising a shampoo or shower gel active base in amount comprised between 85 and 100% by weight, based on the total weight of the perfumed consumer product. The main constituent of the shampoo or a shower gel active base is water or water-based solvents The shampoo shower gel active base may comprise sodium alkylether sulfate, ammonium alkylether sulfates, alkylamphoacetate, cocamidopropyl betaine, cocamide MEA, alkylglucosides and aminoacid based surfactants.

According to a particular embodiment of the invention, the invention's perfumed consumer product is a soap bar comprising a soap active base in amount comprised between 85 and 100% by weight, based on the total weight of the perfumed consumer product. The soap bar active base may comprise salt of a weak acid, typically, a salt of weak acid, which may be a fatty acid and strong base like sodium hydroxide.

Some of the above-mentioned perfumed consumer products may represent an aggressive medium for the invention's composition of matter, so that it may be necessary to protect the latter from premature decomposition, for example by encapsulation or by chemically binding it to another chemical which is suitable to release the invention's ingredient upon a suitable external stimulus, such as an enzyme, light, heat or a change of pH.

The proportions in which the composition of matter according to the invention can be incorporated into the various aforementioned products or compositions vary within a wide range of values. These values are dependent on the nature of the article to be perfumed and on the desired organoleptic effect as well as on the nature of the co-ingredients in a given base when the composition of matter according to the invention are mixed with perfuming co-ingredients, solvents or additives commonly used in the art.

For example, in the case of perfuming compositions, typical concentrations are in the order of 0.001 % to 30 % by weight, or even more, of the composition of matter of the invention based on the weight of the composition into which they are incorporated. In the case of perfumed consumer product, typical concentrations are in the order of 0.0001 % to 10 % by weight, or even more, of the composition of matter of the invention based on the weight of the consumer product into which they are incorporated.

The invention's compositions of matter can be prepared according to a method as described herein-below.

### Examples

The invention will now be described in further detail by way of the following examples, wherein the abbreviations have the usual meaning in the art, the temperatures are indicated in degrees centigrade (°C). NMR spectra were acquired using either a Bruker Avance II Ultrashield 400 plus operating at 400 MHz, (¹H) and 100 MHz (¹³C) or a Bruker Avance III 500 operating at 500 MHz (¹H) and 125 MHz (¹³C) or a Bruker Avance III 600 cryoprobe operating at 600 MHz (¹H) and 150 MHz (¹³C). Spectra were internally referenced relative to tetramethyl silane 0.0 ppm. ¹H NMR signal shifts are expressed in δ ppm, coupling constants (*J*) are expressed in Hz with the following multiplicities: s, singlet; d, doublet; t, triplet; q, quartet; m, multiplet; b, broad (indicating unresolved couplings) and were interpreted using Bruker Topspin software. ¹³C NMR data are expressed in chemical shift δ ppm and hybridization from DEPT 90 and DEPT 135 experiments, C, quaternary (s); CH, methine (d); CH₂, methylene (t); CH₃, methyl (q). Gas chromatography was performed using an Agilent 6850 Instrument, equipped with a DB-23 column (20 M, 0.18 mm, 0.2µm, H₂, 110°C at 3°C/min) with hydrogen as carrier gas, 2.0 mL/min, 1.0 µL injection, with 100:1 split and relative compositions are quoted in % using FID detection.

### Example 1

### Synthesis of the invention's composition of matter

### a) Epoxidation of commercially available Habanolide^{®}

mCPBA (<75% tech. 40.5 g, 1.2 *eq,* 176 mmol), was added in small portions to a stirred solution of commercially available Habanolide^{®} (35.0 g, 147 mmol) in toluene (150 mL), cooled to 5°C in an ice bath. The suspension was stirred for 4 hours at ambient temperature, then diluted with water and the aqueous phase was re extracted with diethyl ether, the organic phase was washed with saturated sodium bicarbonate solution, 10% sodium sulfite solution, dried over anhydrous sodium sulfate, filtered and the solvents were removed *in vacuo* to yield the crude epoxide mixture, 40.1 g. Further purification by Kügelrohr distillation at 135-140°C, 0.8 mbar gave the epoxide mixture, 36.7 g.

### (1SR, 16SR)-5,17-dioxabicyclo[14.1.0]heptadecan-6-one

¹H NMR (500 MHz, CDCl₃):δ 1.13-1.22 (m, 1H), 1.23-1.40 (m, 10H), 1.45-1.53 (m, 2H), 1.65-1.73 (m, 4H), 1.74-1.89 (m, 2H), 2.00 (ddd, J 13.7, 8.7, 5.0, 1H), 2.28-2.41 (m, 2H), 2.64 (dt, *J* 8.7, 2.7, 1H), 2.74 (td, *J* 5.6, 2.1, 1H), 4.13-4.25 (m, 2H) ppm.

¹³C NMR (90 MHz, CDCl₃): δ 25.0 (t), 25.2 (t), 27.3 (t), 27.4 (t), 27.5 (t), 27.9 (t), 28.0 (t), 28.1 (t), 30.8 (t), 34.5 (t), 58.5 (d), 58.6 (d), 62.9 (t), 173.8 (s) ppm.

### (1RS,16RS)-4,17-dioxabicyclo[14.1.0]heptadecan-6-one

¹H NMR (500 MHz, CDCl₃): δ 0-1.54 (m, 15H), 1.67 (bq, *J* 6.8, 2H), 1.77-1.85 (m, 1H),1.89-1.96 (m, 2H), 2.31 (dt, *J* 14.2, 7.1, 1H), 2.41 (dt, *J* 14.2, 6.9, 1H), 2.71 (ddd, *J* 8.1, 3.6, 2.2, 1H), 2.84, (td, *J* 5.5 2.2, 1H), 4.16, (ddd, *J* 11.3, 8.8, 2.6, 1H), 4.32 (ddd, *J* 11.3, 6.7, 3.1, 1H) ppm.

¹³C NMR (125 MHz, CDCl₃): δ 24.3 (t), 24.6 (t), 26.3 (t), 26.4 (t), 26.4 (t), 26.5 (t), 26.9 (t), 27.2 (t), 30.2 (t), 31.6 (t), 34.2 (t), 56.6 (d), 58.6 (d), 61.0 (t), 173.7 (s) ppm.

### (1SR,16RS)-5,17-dioxabicyclo[14.1.0]heptadecan-6-one

¹H NMR (500 MHz, CDCl₃): δ 1.23-1.95 (m, 20H), 2.33 (t, *J* 6.6, 2H), 2.90 -2.97 (m, 2H); 4.14 (ddd, *J* 11.1, 8.1, 3.3, 1H), 4.23 (ddd, *J* 10.9, 6.9, 3.7, 1H) ppm.

¹³C NMR (90 MHz, CDCl₃): δ 24.1 (t), 24.9 (t), 25.1 (t), 26.0 (t), 26.3 (t), 27.1 (t), 27.2 (t), 27.3 (t), 27.8 (t), 34.3 (t), 56.6 (d), 57.1 (d), 63.7 (t), 173.9 (s) ppm.

### (1SR,16RS)-4,17-dioxabicyclo[14.1.0]heptadecan-6-one

¹H NMR (500 MHz, CDCl₃): δ 1.20-1.58 (m, 16 H), 1.60-1.75 (m, 2H), 1.80-1.98 (m, 2H), 2.26-2.38 (m, 2H), 2.92-2.99 (m, 2H), 4.30 (t, *J* 6.9, 2H) ppm.

¹³C NMR (125 MHz, CDCl₃): δ 24.1 (t), 24.6 (t), 26.4 (t), 26.6 (t), 26.6 (t), 26.7 (t), 26.9 (t), 27.0 (t), 27.3 (t), 27.7 (t), 34.4 (t), 54.4 (d), 56.7 (d), 61.8 (t), 173.9 (s) ppm.

### b) Selective hydrolysis of (1RS,16RS)-4,17-dioxabicyclo[14.1.0]heptadecan-6-one and (1SR,16RS)-4,17-dioxabicyclo[14.1.0]heptadecan-6-one

Amberlyst^{®} A-15 (2.0 g, 10% weight) was added to a stirred solution of the epoxide mixture prepared in step a) (20.0 g 78.7 mmol) in ethyl formate (100 mL) and the suspension was stirred at ambient temperature for 18 hours. The suspension was diluted with diethyl ether, washed with saturated sodium bicarbonate solution, brine and the organic phase was dried with anhydrous sodium sulfate, filtered and the solvents were removed *in vacuo* to yield a mixture of (1*SR*,16*SR*)-5,17-dioxabicyclo[14.1.0]heptadecan-6-one, *(1SR,16RS)-5,17-*dioxabicyclo[14.1.0]heptadecan-6-one, *(SR)-13-((RS)-1,3-*dihydroxypropyl)oxacyclotridecan-2-one and *(SR)-13-((SR)-1,3-*dihydroxypropyl)oxacyclotridecan-2-one, 21.2 g. Further purification by filtration through a short plug of silica (18 cm, 400 mL) with ethyl acetate:heptane gradient 5:95 to 15:85 gave a mixture of (1*SR*,16*SR*)-5,17-dioxabicyclo[14.1.0]heptadecan-6-one, (1*SR,*16*RS)-*5,17-dioxabicyclo[14.1.0]heptadecan-6-one, (10.2 g), mixed fractions (5.2 g) and further elution gave a mixture of (*SR*)-13-((*RS*)-1,3-dihydroxypropyl)oxacyclotridecan-2-one and (*SR*)-13-((*SR*)-1,3-dihydroxypropyl)oxacyclotridecan-2-one, (6.25 g). Further purification of the mixture of (1*SR*,16*SR*)-5,17-dioxabicyclo[14.1.0]heptadecan-6-one, (1*SR,*16*RS)-*5,17-dioxabicyclo[14.1.0]heptadecan-6-one (10.2 g) by flash chromatography (column 330 g Puriflash^{®} 40 µM, with a gradient of ethyl acetate:heptane 1:99 to 5:95 as eluent gave (1SR,16SR)-5,17-dioxabicyclo[14.1.0]heptadecan-6-one (5.1 g, as major isomer), further elution gave a mixture of (1*SR*,16*SR*)-5,17-dioxabicyclo[14.1.0]heptadecan-6-one, (1*SR*,16*RS*)-5,17-dioxabicyclo[14.1.0]heptadecan-6-one (58/39, E/Z, 1.95 g) and pure (1*SR*,16*RS*)-5,17-dioxabicyclo[14.1.0]heptadecan-6-one (1.6 g).

### c) Hydrolysis of a mixture comprising as major isomer (1SR,16SR)-5,17-dioxabicyclo[14.1.0]heptadecan-6-one

(1*SR*,16*SR*)-5,17-dioxabicyclo[14.1.0]heptadecan-6-one (major isomer), obtained in step b) (5.1 g, 20 mmol) in THF (70 mL) containing 10% H₂SO₄ (10 mL) was heated at 65°C for 12 hours. The cooled mixture was diluted with saturated sodium bicarbonate solution and extracted with diethyl ether, the organic phase was washed with brine, dried over anhydrous sodium sulfate, filtered and the solvents removed *in vacuo* to yield the crude diol, 4.7 g, which was used directly in the next step without further purification.

### (12RS,13SR)-12,13-dihydroxyoxacyclohexadecan-2-one

¹H NMR (500 MHz, CDCl₃):δ 1.25-1.48 (m, 16H), 1.57-1.94 (m, 7H), 2.34 (*m*-11, *J* 6.8, 4H), 3.41-3.51 (m, 2H), 4.14-4.21 (m, 2H) ppm.

¹³C NMR (125 MHz, CDCl₃): δ 22.1 (t), 24.6 (t), 24.9 (t), 25.7 (t), 26.2 (t), 26.5 (t), 27.0 (t), 27.8 (t), 29.7 (t), 31.5 (t), 34.4 (t), 63.8 (t), 73.6 (d), 74.5 (d), 174.1 (s) ppm.

### d) Conversion of mixture of diols obtained in step c) in ortho esters and Elimination

A suspension of citric acid (170 mg, 0.9 mmol), the mixture of diols prepared in step c) (4.8 g, 17.6 mmol) trimethyl orthoformate (9.5 g, 89 mmol) was heated at 70°C for 45 minutes using a rotary evaporator to form the intermediate orthoesters. The solvents were removed *in vacuo* and the residue was dissolved in acetic anhydride (14.0 g, 140 mmol) and the mixture was heated in 3 x 1.5 g batches using an Anton Parr Monowave 50, at 150°C for 60 minutes then cooled. The batched were combined, diluted with diethyl ether (50 mL) and washed with sodium carbonate solution and then saturated sodium bicarbonate solution, brine, dried over anhydrous sodium sulfate, filtered and the solvents removed *in vacuo* to give the crude invention `s composition of matter, 3.7 g. Further purification by flash chromatography (220 g, Puriflash^{®} column 40 µM Silica) with heptane:ethyl acetate (99:1 to 97:3) as eluant gave 2.4 g of pure invention 's composition of matter, which was Kügelrohr distilled (130°C, 0.8 mbar) and gave pure invention's composition of matter comprising 97% w/w of (*Z*)-oxacyclohexadec-12-en-2-one, about 1.1% w/w of (*E*)-oxacyclohexadec-12-en-2-one and about 1.6% w/w of (*Z*)-oxacyclohexadec-13-en-2-one, 2.2 g.

### (Z)-oxacyclohexadec-12-en-2-one

¹H NMR (500 MHz, CDCl₃): δ 1.20-1.42 (m, 12H), 1.64-1.76 (m, 4H), 2.04 (q, *J* 6.9, 2H), 2.14-2-22 (m, 2H), 2.30 (m, 2H), 4.14 (bt, *J* 5.4, 2H), 5.30 -5.44 (m, 2H) ppm.

¹³C NMR (90 MHz, CDCl₃): δ 24.2 (t), 24.7 (t), 26.2 (t), 26.3 (t), 26.9 (t), 27.0 (t), 27.6 (t), 29.2 (t), 34.2 (t), 63.6 (t), 128.6 (d), 131.0 (d), 173.9 (s) ppm.

### Example 2

### Synthesis of the invention's composition of matter

Peracetic acid (39% in acetic acid, 120 mmol, 23.4 g) was added slowly dropwise over 2 hours to a stirred quantity of commercially available Habanolide^{®} (25.0 g, 97%, 105 mmol) heated at 50°C. 10% H₂SO₄ (5 g) was then added slowly dropwise and heating continued at 50°C for a further 2 hours. The mixture was cooled and diluted with ethyl acetate and 10% sodium sulfite solution, the aqueous phase re-extracted with ethyl acetate, then the organic phase was washed with saturated sodium bicarbonate solution, brine, dried over anhydrous MgSO₄, filtered and the solvents removed *in vacuo* to yield the crude mixture of diols, 29.2 g. This diol mixture was purified by filtration through a short plug of silica (200 mL) with heptane:ethyl acetate (1:9) (2 x 250 mL) then heptane:ethyl acetate (1:6) (2 x 250 mL), then heptane:ethyl acetate (3:7) (500 mL) and finally heptane:ethyl acetate (1:1) and then pure ethyl acetate (500 mL) gave the diol mixture, as an oil that slowly crystallized. 11.0 g. A portion of this diol mixture (5.0 g, 18.4 mmol) was dissolved in dichloromethane (20 mL) and rac-Camphor sulfonic acid (250 mg, 1,07 mmol cat.) was added and the mixture was stirred at ambient temperature for 15 hours. The mixture was diluted with diethyl ether, washed with saturated sodium bicarbonate solution, brine, dried over anhydrous sodium sulfate, filtered and the solvents removed *in vacuo* to yield the desired diol mixture, 5.3 g.

A solution of C-24 dicarboxylic acid (455 mg, 1.14 mmol), the diol mixture prepared above (5.3 g, 19.5 mmol) trimethyl orthoformate (2.5 g, 23.5 mmol) was heated at 70°C for 45 minutes using a Kügelrohr flask to form the intermediate orthoesters. The solvents were removed *in vacuo* and the residue was put under vacuum (0,8 mbar) and heated slowly to 155°C-165°C to give the crude invention's composition of matter, 4.2 g. Further purification by flash chromatography (220 g, Puriflash^{®} column 40 mM Silica) with heptane:ethyl acetate (99:1 to 97:3) as eluant gave 2.1 g of pure invention's composition of matter, which was Kügelrohr distilled (130°C, 0.8 mbar) and gave pure invention's composition of matter comprising 61.5% w/w of (*Z*)-oxacyclohexadec-12-en-2-one, about 6.1% w/w of (*E*)-oxacyclohexadec-12-en-2-one, about 3.4% w/w of (*E*)-oxacyclohexadec-13-en-2-one and about 28.7% w/w of (*Z*)-oxacyclohexadec-13-en-2-one, 2.0 g.

### Example 3

### Synthesis of the invention's composition of matter

### a) Selective hydrolysis of (1SR,16RS)-5,17-dioxabicyclo[14.1.0]heptadecan-6-one and (1SR,16RS)-4,17-dioxabicyclo[14.1.0]heptadecan-6-one

Dilute sulfuric acid (10 mL, 5%, 0.05 eq) was added to a stirred solution of the epoxide mixture prepared in Example 1 a) (28.0 g, 110 mmol) in THF (100 mL) and stirred for 48 hours at ambient temperature. The reaction mixture was diluted with MTBE and then washed with saturated sodium bicarbonate solution, brine, dried over anhydrous sodium sulfate, filtered and the solvents removed *in vacuo* to yield a mixture of (12SR,13SR)-12,13-dihydroxyoxacyclohexadecan-2-one, (13RS,14RS)-13,14-dihydroxyoxacyclohexadecan-2-one, (1SR,16SR)-5,17-dioxabicyclo[14.1.0]heptadecan-6-one and (1RS,16RS)-4,17-dioxabicyclo[14.1.0]heptadecan-6-one, 20.9 g. Further purification by filtration through a small plug of silica (15 cm, 200 mL) and heptane : ethyl acetate (99:1 to 95:5) as eluant (100 mL fractions) gave the (1SR,16SR)-5,17-dioxabicyclo[14.1.0]heptadecan-6-one and (1*RS*,16*RS*)-4,17-dioxabicyclo[14.1.0]heptadecan-6-one, 7.3 g,

### (1SR,16SR)-5,17-dioxabicyclo[14.1.0]heptadecan-6-one

¹H NMR (500 MHz, CDCl₃): δ 1.13-1.22 (m, 1H), 1.23-1.40 (m, 10H), 1.45-1.53 (m, 2H), 1.65-1.73 (m, 4H), 1.74-1.89 (m, 2H), 2.00 (ddd, *J* 13.7, 8.7, 5.0, 1H), 2.28-2.41 (m, 2H), 2.64 (dt, *J* 8.7, 2.7, 1H), 2.74 (td, *J* 5.6, 2.1, 1H), 4.13-4.25 (m, 2H) ppm.

¹³C NMR (90 MHz, CDCl₃): δ 25.0 (t), 25.2 (t), 27.3 (t), 27.4 (t), 27.5 (t), 27.9 (t), 28.0 (t), 28.1 (t), 30.8 (t), 34.5 (t), 58.5 (d), 58.6 (d), 62.9 (t), 173.8 (s) ppm.

### (1RS,16RS)-4,17-dioxabicyclo[14.1.0]heptadecan-6-one

¹H NMR (500 MHz, CDCl₃): δ 0-1.54 (m, 15H), 1.67 (bq, *J* 6.8, 2H), 1.77-1.85 (m, 1H), 1.89-1.96 (m, 2H), 2.31 (dt, *J* 14.2, 7.1, 1H), 2.41 (dt, *J* 14.2, 6.9, 1H), 2.71 (ddd, *J* 8.1, 3.6, 2.2, 1H), 2.84, (td, *J* 5.5 2.2, 1H), 4.16, (ddd, *J* 11.3, 8.8, 2.6, 1H), 4.32 (ddd, *J* 11.3, 6.7, 3.1, 1H) ppm.

¹³C NMR (125 MHz, CDCl₃): δ 24.3 (t), 24.6 (t), 26.3 (t), 26.4 (t), 26.4 (t), 26.5 (t), 26.9 (t), 27.2 (t), 30.2 (t), 31.6 (t), 34.2 (t), 56.6 (d), 58.6 (d), 61.0 (t), 173.7 (s) ppm.

### b) Selective hydrolysis of a mixture comprising (1SR,16SR)-5,17-dioxabicyclo[14.1.0]heptadecan-6-one and (IRS,16RS)-4,17-dioxabicyclo[14.1.0]heptadecan-6-one

Amberlyst^{®} A-15 (0.7g, 10% weight) was added to a stirred solution of the mixture comprising (1*SR*,16*SR*)-5,17-dioxabicyclo[14.1.0]heptadecan-6-one and (1*RS*,16*RS*)-4,17-dioxabicyclo[14.1.0]heptadecan-6-one prepared in previous step (12:13 isomer, 65:35, 7.3 g, 28.7 mmol) in ethyl formate (20 mL) and the suspension was stirred at ambient temperature for 15 hours. The reaction mixture was diluted with diethyl ether, washed with saturated sodium bicarbonate solution, brine and the organic phase was dried with anhydrous sodium sulfate, filtered and the solvents were removed *in vacuo* to yield a mixture of the (1*SR*,16*SR*)-5,17-dioxabicyclo[14.1.0]heptadecan-6-one and (*SR*)-13-((*RS*)-1,3-dihydroxypropyl)oxacyclotridecan-2-one, 8.1 g. Further purification by filtration through a short plug of silica (10 cm, 200 mL) with ethyl acetate:heptane gradient 5:95 to 50:50 gave 4.7 g (1*SR*,16*SR*)-5,17-dioxabicyclo[14.1.0]heptadecan-6-one and further elution gave the (*SR*)-13-((*RS*)-1,3-dihydroxypropyl)oxacyclotridecan-2-one, 2.35 g.

### (SR)-13-((RS)-1,3-dihydroxypropyl)oxacyclotridecan-2-one

¹H NMR (500 MHz, CDCl₃): δ 1.18-1.87 (m, 15 H), 2.03-2.11 (m, 1H), 2.31-2-37 (m, 2H), 2.41-2.47 (m, 1H), 2.85 (bs, 2H), 3.61-3.89 (m, 4H), 4.89 (td, *J* 7.9, 4.1, 1H) ppm.

¹³C NMR (125 MHz, CDCl₃): δ 21.1 (t), 23.6 (t), 24.0 (t), 24.8 (t), 25.5 (t), 25.8 (t), 25.9 (t), 26.3 (t), 31.1 (t), 34.1 (t), 34.6 (t), 58.6 (t), 72.2 (d), 74.3 (d), 174.3 (s) ppm.

### c) Isomerisation of (SR)-13-((RS)-1,3-dihydroxypropyl)oxacyclotridecan-2-one

Rac-camphor sulfonic acid (200 mg, 0.8 mmol) was added to a solution of (*SR*)-13-((*RS*)-1,3-dihydroxypropyl)oxacyclotridecan-2-one, (2.35 g, 8.6 mmol) prepared in previous step, in dichloromethane (20 mL) and the mixture was stirred at ambient temperature for 15 hours. The mixture was diluted with diethyl ether, washed with saturated sodium bicarbonate solution, brine, dried over anhydrous sodium sulfate, filtered and the solvents removed *in vacuo* to yield the desired (13*RS*,14*SR*)-13,14-dihydroxyoxacyclohexadecan-2-one and (13*RS*,14*RS*)-13,14-dihydroxyoxacyclohexadecan-2-one, 2.0 g.

### (13RS,14SR)-13,14-dihydroxyoxacyclohexadecan-2-one

¹H NMR (500 MHz, CDCl₃): δ 1.20-1.86 (m, 19H), 2.20-2.49 (m, 3H), 3.76 (dt, *J* 10.3, 2.9, 1H), 3.85 (ddd, *J* 8.6, 5.5, 3.2, 1H), 4.29-4.41 (m, 2H) ppm.

¹³C NMR (125 MHz, CDCl₃): δ 23.1 (t), 24.5 (t), 25.3 (t), 26.0 (t), 26.3 (t), 26.4 (t), 27.0 (t), 27.3 (t), 28.9 (t), 32.2 (t), 34.5 (t), 60.7 (t), 69.1 (d), 74.0 (d), 174.3 (s) ppm.

### d) Formation of orthoester and elimination

### i) One pot process with citric acid

The diol prepared in step c) (1.0 g, 3.67 mmol) was dissolved in trimethyl orthoformate (2.0 g, 18.9 mmol) and citric acid (35 mg, 0.18 mmol) was added and the mixture was heated in an Anton Parr Monowave 50 at 175°C for 2 hours then cooled. The reaction mixture was diluted with diethyl ether, and saturated sodium bicarbonate solution. The aqueous phase was re extracted with diethyl ether, then the combined organic phase was washed with saturated sodium bicarbonate solution then brine, dried over anhydrous sodium sulfate, filtered and the solvents removed *in vacuo* to yield the crude invention's composition of matter, 0.9 g, still containing *ca.* 20% orthoesters. Further purification by chromatography using a Puriflash^{®} (40 g cartridge 30 mM) with 99:1 to 97:3 heptane:ethyl acetate as eluent gave the invention's composition of, 0.6 g which was further purified by Kügelrohr distillation (145°C at 0.5 mbar) and gave pure the invention's composition of matter comprising about 5.4% w/w of (*Z*)-oxacyclohexadec-12-en-2-one, about 0.3% w/w of (*E*)-oxacyclohexadec-12-en-2-one, about 0.6% w/w of (*E*)-oxacyclohexadec-13-en-2-one and about 93.7% w/w of (*Z*)-oxacyclohexadec- 13-en-2-one, 0.45 g.

### (Z)-oxacyclohexadec-13-en-2-one

¹H NMR (500 MHz, CDCl₃): δ 1.62-1.70 (m, 14H), 1.62-1.70 (m, 2 H), 2.06 (bq, *J* 7, 2H), 2.29 (t, *J* 7.2, 2H), 2.41 (bq, *J* 6.4, 1H), 4.12 (t, *J* 6.6, 2H), 5.30-5.37 (m, 1H), 5.50-5.57 (m, 1H) ppm.

¹³C NMR (90 MHz, CDCl₃): δ 24.1 (t), 25.8 (t), 26.3 (t), 26.5 (t), 26.6 (t), 26.7 (t), 26.9 (t), 27.0 (t), 27.2 (t), 28.1 (t), 34.5 (t), 63.9 (t), 125.4 (d), 132.7 (d), 174.1 (s) ppm.

### ii) Alternative method: formation of orthoester, then thermolysis with acetic anhydride

The diol prepared in step c) (1.0 g, 3.67 mmol) was dissolved in trimethyl orthoformate (2.0 g, 18.9 mmol) and citric acid (35 mg, 0.18 mmol) was heated at 75°C for 45 minutes then cooled and the excess orthoformate was evaporated *in vacuo,* to give the crude ortho ester.

### (3aSR,17aRS)-2-methoxytetradecahydro-7H-[1,3]dioxolo[4,5-d][1]oxacyclohexadecin-7-one

¹H NMR (500 MHz, *d*-5 Pyridine): δ 1.05-1.40 (m, 15H), 1.50-1.80 (m, 6H), 2.33 (t, *J* 6.2, 2H), 3.36 (s, 3H), 4.23-4.60 (m, 3H), 5.96 (s, 1H) ppm. (Minor isomers present at 3.38, 3.35, 3.33 and 6.02, 6.00, 5.92 ppm.)

¹³C NMR (125 MHz, *d*-5 Pyridine): δ 23.4 (t), 25.2 (t), 25.3 (t), 26.4 (t), 27.0, 27.3, 27.4, 27.5, 27.6, 27.7 (t), 28.4, 28.8 (t), 29.4, 29.9 (t), 34.7, 34.8 (t), 51.3, 51.6 (q), 61.3, (t), 75.4, (d), 77.3, (d), 78.3 (d) 115.1, (d), 173.4, 173.3 (s) ppm; (Minor isomers at 116.4, 116.2, 116.0 and 60.9, 60.8, 74.7 ppm.)

The crude orthoester was used without further purification and dissolved in acetic anhydride (2.0 g, 19.6 mmol) was heated at 150°C for 1 hour then cooled. The mixture was diluted with diethyl ether, and water and stirred for 1 hour at ambient temperature. The aqueous phase was re extracted with diethyl ether, then the combined organic phase was washed with saturated sodium bicarbonate solution then brine, dried over anhydrous sodium sulfate, filtered and the solvents removed *in vacuo* to yield the crude invention's composition of matter, 0.8 g. Further purification by chromatography using a Puriflash^{®} (40 g cartridge 30 mM) with 99:1 to 97:3 heptane:ethyl acetate as eluent gave the invention's composition of matter, 0.5 g which was further purified by Kügelrohr distillation (145°C at 0.5 mbar) and gave pure about 5.5% w/w of (Z)-oxacyclohexadec-12-en-2-one, about 0.2% w/w of (E)-oxacyclohexadec-12-en-2-one, about 0.6% w/w of (E)-oxacyclohexadec-13-en-2-one and about 93.7% w/w of (Z)-oxacyclohexadec-13-en-2-one, 0.4 g.

### Example 4

### Preparation of a perfuming composition

A perfuming composition for fine fragrance was prepared by admixing the following ingredients:

| **Ingredient name** | **Parts by weight** |
|---|---|
| 10% * Absinthe | 80 |
| (E)-2-methoxy-4-(1-propenyl)phenyl acetate | 80 |
| (+-)-1,5-dimethyl-1-vinyl-4-hexenyl acetate | 200 |
| 1 % * (+-)-2-methylundecanal | 40 |
| 10% * (-)-(3aR,5aS,9aS,9bR)-3a,6,6,9a-tetramethyldodecahydronaphtho[2,1-b]furan | 60 |
| 1,2,3,5,6,7-hexahydro-1,1,2,3,3-pentamethyl-4-indenone | 100 |
| Cedarwood oil | 140 |
| Lemon oil | 600 |
| (+-)-3,7-dimethyl-6-octen-1-ol | 20 |
| Coriander oil | 40 |
| Coumarin | 100 |
| 10% * (+-)-(2E)-1-(2,6,6-trimethyl-2-cyclohexen-1-yl)-2-buten-1-one | 40 |
| (-)-(2E)-2-ethyl-4-[(1R)-2,2,3-trimethyl-3-cyclopenten-1-yl]-2-buten-1-ol | 80 |
| (+-)-2,6-dimethyl-7-octen-2-ol | 200 |
| Elemi | 100 |
| Elemi resin | 800 |
| 2-methoxy-4-(2-propen-1-yl)phenol | 20 |
| Gaiac | 160 |
| (E)-3,7-dimethyl-2,6-octadien-1-ol | 10 |
| Gurjun balsam | 400 |
| Methyl 2-((1RS,2RS)-3-oxo-2-pentylcyclopentyl)acetate | 2200 |
| 1-(octahydro-2,3,8,8-tetramethyl-2-naphtalenyl)-1-ethanone | 2000 |
| (+-)-3,7-dimethyl-1,6-octadien-3-ol | 200 |
| Nutmeg absolute pure and nat | 20 |
| Patchouli oil | 100 |
| Pimenta berry oil | 10 |
| Black pepper steam dist. | 60 |
| 5-(2,2,3-Trimethyl-3-cyclopentenyl)-3-methylpentan-2-ol | 10 |
| Sage oil | 20 |
| (+-)-alpha-terpineol | 10 |
| 4-hydroxy-3-methoxybenzaldehyde | 60 |
| 10% * 2,4-Dimethyl-3-cyclohexene-1-carbaldehyde) | 40 |
| | **8000** |

| | |
|---|---|
| * in dipropyleneglycol | |

The addition of 2000 parts by weight of the composition of matter described in Example 1 to the above-described fine fragrance composition imparted to the latter a musky character in the direction of nitro musk with a strong powdery character. The invention's composition of matter blends particularly well with powdery elements such as Coumarin and oriental notes such as vanillin.

The addition of 2000 parts by weight of the composition of matter described in Example 2 to the above-described fine fragrance composition imparted to the latter a musky character in the direction of nitro musk with a strong powdery character as well as creamy and oriental note. The invention's composition of matter blends particularly well with powdery elements such as Coumarin, oriental notes such as vanillin and woody-sandalwood elements such as (-)-(2E)-2-ethyl-4-[(1R)-2,2,3-trimethyl-3-cyclopenten-1-yl]-2-buten-1-ol and 5-(2,2,3-Trimethyl-3-cyclopentenyl)-3-methylpentan-2-ol.

The addition of the same amount of Habanolide^{®} also imparted a musky character but in the direction of macrocyclic musk but with woody aspect. The composition obtained by said addition is devoid of powdery, creamy and oriental character. Habanolide^{®} blends particularly well with woody-cedar elements such as Cedar wood oil and 1-(octahydro-2,3,8,8-tetramethyl-2-naphtalenyl)-1-ethanone.

The addition of the invention's compositions of matter to the above-described fine fragrance composition provides more volume to the composition. Said effect is also obtained with Habanolide^{®} but at a lower extent.

### Example 5

### Preparation of a eau de toilette comprising the invention's composition of matter

The eau de toilette was prepared by adding 12% by weight, relative to the total weight of the eau de toilette, of the invention's composition of example 4 into ethanol.

### Example 6

### Preparation of a liquid detergent comprising the invention's composition of matter

**Table 1: Composition of the liquid detergent formulation**

| **Ingredients** | **Concentration [wt%]** |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium hydroxide | 9.5 |
| Properase L⁴⁾ | 0.2 |
| Puradax EG L⁴⁾ | 0.2 |
| Purastar ST L⁴⁾ | 0.2 |
| Acrylates/Steareth-20 Methacrylate structuring Crosspolymer⁵⁾ | 6 |
| Deionized Water | 27.4 |

| | |
|---|---|
| 1) Hostapur SAS 60; Origin: Clariant 2) Edenor K 12-18; Origin: Cognis 3) Genapol LA 070; Origin: Clariant 4) Origin: Genencor International 5) Aculyn 88; Origin: Dow Chemical | |

The liquid detergent is prepared by adding 0.5 to 1.5 % by weight, relative to the total weight of the liquid detergent, of the invention's composition of example 4 into the unperfumed liquid detergent formulation of Table 1 under gentle shaking.

### Example 7

### Preparation of a fabric softener comprising the invention's composition of matter

**Table 2: Composition of the softener formulation**

| **Ingredient** | **Concentration [wt%]** |
|---|---|
| Methyl bis[ethyl (tallowate)]-2-hydroxyethyl ammonium methyl sulfate¹⁾ | 12.20 |
| 1,2-benzisothiazolin-3-one²⁾ | 0.04 |
| CaCl₂ (10% aqueous solution) | 0.40 |
| Water | 87.36 |

| | |
|---|---|
| 1) Stepantex VL90 A Diester Quat; Origin: Stepan 2) Proxel GXL; Origin: Arch | |

The softener is prepared by weighting Methyl bis[ethyl (tallowate)]-2- hydroxyethyl ammonium methyl sulfate which was heated at 65°C. Then Water and 1,2-benzisothiazolin-3-one are placed in the reactor and are heated at 65°C under stirring. To the above mixture is added Methyl bis[ethyl (tallowate)]-2- hydroxyethyl ammonium methyl sulfate. The mixture is stirred 15 minutes and CaCl₂ is added. Then 0.5 to 2% by weight, relative to the total weight of the softener, of the invention's composition of example 4 is added. The mixture is stirred 15 minutes and is cooled down to room temperature under stirring (viscosity measure : result 35 +/- 5 mPas. (shear rate 106 sec-1)).

### Example 8

### Preparation of a transparent isotropic shampoo comprising the invention's composition of matter

**Table 3: Composition of the transparent isotropic shampoo formulation**

| **Phases** | **Ingredients** | **Concentration [wt%]** |
|---|---|---|
| **A** | Water deionized | 44.4 |
| | Polyquaternium-10 ¹⁾ | 0.3 |
| | Glycerin 85% ²⁾ | 1 |
| | DMDM Hydantoin ³⁾ | 0.2 |
| **B** | Sodium Laureth Sulfate ⁴⁾ | 28 |
| | Cocamidopropyl Betaine ⁵⁾ | 3.2 |
| | Disodium Cocoamphodiacetate ⁶⁾ | 4 |
| | Ethoxy (20) Stearyl Alcohol ⁶⁾ | 1 |
| **C** | Sodium Laureth Sulfate ⁴⁾ | 3 |
| | Glyceryl Laureate ⁷⁾ | 0.2 |
| **D** | Water deionized | 1 |
| | Sodium Methylparaben ⁸⁾ | 0.1 |
| **E** | Sodium Chloride 10% aqueous sol. | 15 |
| | Citric acid 10% aqueous sol. till pH 5.5-6 | q.s. |

| | | |
|---|---|---|
| 1) Ucare Polymer JR-400, Origin: Noveon 2) Origin: Schweizerhall 3) Glydant, Origin: Lonza 4) Texapon NSO IS, Origin: Cognis 5) Tego Betain F 50, Origin: Evonik 6) Amphotensid GB 2009, Origin: Zschimmer & Schwarz 7) Monomuls 90 L-12, Origin: Gruenau 8) Nipagin Monosodium, Origin: NIPA | | |

The shampoo is prepared by dispersed in water Polyquaternium-10. The remaining ingredients of phase A are mixed separately by addition of one after the other while mixing well after each adjunction. This pre-mix is added to the Polyquaternium-10 dispersion and mixed for another 5 min. Then, the premixed phase B and the premixed Phase C are added (Monomuls 90L-12 is heated to melt in Texapon NSO IS) while agitating. Phase D and Phase E are added while agitating. PH is adjusted with citric acid solution till pH: 5.5 - 6.0 leading to an unperfumed shampoo formulae.

The perfumed shampoo is prepared by adding 0.4 to 0.8% by weight, relative to the total weight of the shampoo, of the invention's composition of example 4 into the unperfumed shampoo formulation of Table 3 under gentle shaking.

### Example 9

### Preparation of a structured shower gel comprising the invention's composition of matter

**Table 4: Composition of the shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 1) EDETA B POWDER; trademark and origin: BASF 2) CARBOPOL AQUA SF-1 POLYMER; trademark and origin: NOVEON 3) ZETESOL AO 328 U; trademark and origin: ZSCHIMMER & SCHWARZ 4) TEGO-BETAIN F 50; trademark and origin: GOLDSCHMIDT 5) KATHON CG; trademark and origin: ROHM & HASS | |

The shower gel is prepared by adding 0.5 to 1.5% by weight, relative to the total weight of the shower gel, of the invention's composition of example 4 into the unperfumed shower gel formulation of Table 4 under gentle shaking.

### Example 10

### Preparation of a transparent shower gel comprising the invention's composition of matter

**Table 5: Composition of the transparent shower gel formulation**

| **Ingredients** | **Concentration (% wt)** |
|---|---|
| WATER deionized | 52.40 |
| Tetrasodium EDTA ¹⁾ | 0.10 |
| Sodium Benzoate | 0.50 |
| Propylene Glycol | 2.00 |
| Sodium C12-C15 Pareth Sulfate ²⁾ | 35.00 |
| Cocamidopropyl Betaine³⁾ | 8.00 |
| Polyquaternium-7⁴⁾ | 0.20 |
| Citric Acid (40%) | 1.00 |
| Sodium Chloride | 0.80 |

| | |
|---|---|
| 1) EDETA B POWDER; trademark and origin: BASF 2) ZETESOL AO 328 U; trademark and origin: ZSCHIMMER & SCHWARZ 3) TEGO-BETAIN F 50; trademark and origin: GOLDSCHMIDT 4) MERQUAT 550; trademark and origin: LUBRIZOL | |

The transparent shower gel is prepared by adding 0.5 to 1.5% by weight, relative to the total weight of the shower gel, of the invention's composition of example 4 into the unperfumed shower gel formulation of Table 5 under gentle shaking.

### Example 11

### Preparation of a milky shower gel comprising the invention's composition of matter

**Table 6: Composition of the milky shower gel formulation**

| **Ingredients** | **Concentration (% wt)** |
|---|---|
| WATER deionized | 50.950 |
| Tetrasodium EDTA ¹⁾ | 0.050 |
| Sodium Benzoate | 0.500 |
| Glycerin 86% | 3.500 |
| Sodium Laureth Sulfate ²⁾ | 27.000 |
| Polyquaternium-7³⁾ | 1.000 |
| Coco-Betaine⁴⁾ | 6.000 |
| PEG-120 Methyl Glucose trioleate⁵⁾ | 1.000 |
| Citric Acid (40%) | 1.000 |
| Glycol Distearate & Laureth-4 & Cocamidopropyl Betaine⁶⁾ | 3.000 |
| Sodium Chloride 20% | 5.000 |
| PEG-40 Hydrogenated Castor Oil⁷⁾ | 1.000 |

| | |
|---|---|
| 1) EDETA B POWDER; trademark and origin: BASF 2) Texapon NSO IS; trademark and origin: COGNIS 3) MERQUAT 550; trademark and origin: LUBRIZOL 4) DEHYTON AB-30; trademark and origin: COGNIS 5) GLUCAMATE LT; trademark and origin: LUBRIZOL 6) EUPERLAN PK 3000 AM; trademark and origin: COGNIS 7) CREMOPHOR RH 40; trademark and origin: BASF | |

The transparent shower gel is prepared by adding 0.5 to 1.5% by weight, relative to the total weight of the shower gel, of the invention's composition of example 4 into the unperfumed shower gel formulation of Table 6 under gentle shaking.

### Example 12

### Preparation of a pearly shampoo comprising the invention's composition of matter

**Table 7: Composition of the pearly isotropic_shampoo formulation**

| **Phases** | **Ingredients** | **Concentration (% wt)** |
|---|---|---|
| **A** | Water deionized | 45.97 |
| | Tetrasodium EDTA ¹⁾ | 0.05 |
| | Guar Hydroxypropyltrimonium Chloride ²⁾ | 0.05 |
| | Polyquaternium-10 ³⁾ | 0.075 |
| **B** | NaOH 10% aqueous sol. | 0.3 |
| **C** | Ammonium Lauryl Sulfate ⁴⁾ | 34 |
| | Ammonium Laureth Sulfate ⁵⁾ | 9.25 |
| | Cocamidopropyl Betaine ⁶⁾ | 2 |
| | Dimethicone (&) C12-13 Pareth-4 (&) C12-13 Pareth-23 (&) Salicylic Acid ⁷⁾ | 2.5 |
| **D** | Cetyl Alcohol ⁸⁾ | 1.2 |
| | Cocamide MEA ⁹⁾ | 1.5 |
| | Glycol Distearate ¹⁰⁾ | 2 |
| **E** | Methylchloroisothiazolinone & Methylisothiazolinone 11) | 0.1 |
| | D-Panthenol 75% ¹²⁾ | 0.1 |
| | Water deionized | 0.3 |
| **F** | Sodium Chloride 25% aqueous sol. | 0.6 |

| | | |
|---|---|---|
| 1) EDETA B Powder, Origin: BASF 2) Jaguar C14 S, Origin: Rhodia 3) Ucare Polymer JR-400, Origin: Noveon 4) Sulfetal LA B-E, Origin: Zschimmer & Schwarz 5) Zetesol LA, Origin: Zschimmer & Schwarz 6) Tego Betain F 50, Origin: Evonik 7) Xiameter MEM-1691, Origin: Dow Corning 8) Lanette 16, Origin: BASF 9) Comperlan 100, Origin: Cognis 10) Cutina AGS, Origin: Cognis 11) Kathon CG, Origin: Rohm & Haas 12) D-Panthenol, Origin: Roche | | |

The shampoo is prepared by dispersed in water and Tetrasodium EDTA, Guar Hydroxypropyltrimonium Chloride and Polyquaternium-10. NaOH 10% solution (Phase B) is added once Phase A is homogeneous. Then, the premixed Phase C is added. and mixture is heated to 75°C. Phase D ingredients are added and mixed till homogeneous. The mixture is cooled down. At 45°C, Phase E ingredients are added while mixing. Final viscosity is adjusted with 25% NaCl solution and pH of 5.5-6 is adjusted with 10% NaOH solution.

The perfumed pearly shampoo is prepared by adding 0.4 to 0.8% by weight, relative to the total weight of the shampoo, of the invention's composition of example 4 into the unperfumed shampoo formulation of Table 7 under gentle shaking.

### Example 13

### Preparation of a structured shower gel comprising the invention's composition of matter

**Table 8: Composition of the milky shower gel formulation**

| **Ingredients** | **Amount (% wt)** |
|---|---|
| WATER deionised | 49.350 |
| Tetrasodium EDTA ¹⁾ | 0.050 |
| Acrylates Copolymer²⁾ | 6.000 |
| Sodium C12-C15 Pareth Sulfate ³⁾ | 35.000 |
| Sodium Hydroxide 20% aqueous solution | 1.000 |
| Cocamidopropyl Betaine⁴⁾ | 8.000 |
| Methylchloroisothiazolinone and Methylisothiazolinone⁵⁾ | 0.100 |
| Citric Acid (40%) | 0.500 |

| | |
|---|---|
| 6) EDETA B POWDER; trademark and origin: BASF 7) CARBOPOL AQUA SF-1 POLYMER; trademark and origin: NOVEON 8) ZETESOL AO 328 U; trademark and origin: ZSCHIMMER & SCHWARZ 9) TEGO-BETAIN F 50; trademark and origin: GOLDSCHMIDT 10) KATHON CG; tradeark and origin: ROHM & HASS | |

The transparent shower gel is prepared by adding 0.5 to 1.5% by weight, relative to the total weight of the shower gel, of the invention's composition of example 4 into the unperfumed shower gel formulation of Table 8 under gentle shaking.

### Example 14

### Preparation of anhydrous antiperspirant spray formulations comprising the invention's composition of matter

**Table 9: Composition of the anhydrous antiperspirant spray formulation**

| **Ingredients** | **Amount (% wt)** |
|---|---|
| Cyclomethicone ⁽¹⁾ | 53.0 |
| Isopropyl myristate | 9.0 |
| Silica ⁽²⁾ | 1.0 |
| Quaternium-18-hectorite ⁽³⁾ | 3.3 |
| Aluminium chlorohydrate ⁽⁴⁾ | 32.7 |
| Perfume oil | 1 |

| | |
|---|---|
| ⁽¹⁾ Dow Corning^{®} 345 Fluid; origin: Dow Corning ⁽²⁾ Aerosil^{®} 200 ; origin: Evonik ⁽³⁾ Bentone^{®} 38; origin: Elementis Specialities ⁽⁴⁾ Micro Dry Ultrafine; origin: Reheis | |

Anhydrous antiperspirant spray formulation is prepared by using a high speed stirrer. Silica and Quaternium-18-hectorite are added to the mixture of isopropyl myristate and cyclomethicone. Once completely swollen, aluminium chlorohydrate is added portion-wise under stirring until the mixture becomes homogeneous and without lumps. Then a perfume oil being the invention's composition of example 4 is added.

### Example 15

### Preparation of deodorant spray emulsion formulations comprising the invention's composition of matter

**Table 10: Composition of deodorant spray emulsion formulation**

| **Ingredients** | **Amount (% wt)** |
|---|---|
| Ethanol (95%) | 89.25 |
| Triclosan ⁽¹⁾ | 0.25 |
| Isopropyl myristate | 9.00 |
| Invention's composition of Example 4 | 1.5 |

| | |
|---|---|
| ⁽¹⁾ Irgasan^{®} DP 300; origin: BASF ⁽²⁾ mixture of Compounds 2a/2b ca. 45:55 | |

Deodorant spray emulsion formulation is prepared by mixing and dissolving all the ingredients according to the sequence of Table 10. Aerosol cans are filled, and the propellant is crimped and added. Aerosol filling: 40% active solution 60% propane / butane (2.5 bar).

### Example 16

### Preparation of deodorant stick formulations comprising the invention's composition of matter

**Table 11: Composition of Deodorant stick formulation**

| **Phase** | **Ingredients** | **Amount (% wt)** |
|---|---|---|
| A | Stearic acid | 5.00 |
| | 1,2-Propylene glycol | 41.45 |
| | Sodium hydroxide (20% aqueous solution) | 4.20 |
| | Water | 30.00 |
| | Tetrasodium EDTA ⁽¹⁾ | 0.10 |
| | Ceteareth-25 ⁽²⁾ | 1.50 |
| | PPG-3 Myristyl ether ⁽³⁾ | 1.50 |
| B | 1,2-Propylene glycol | 15.00 |
| | Triclosan ⁽⁴⁾ | 0.25 |
| C | Perfume oil | 1 |

| | | |
|---|---|---|
| ⁽¹⁾ Edeta^{®} B Power; origin: BASF ⁽²⁾ Cremophor^{®} A25; origin: BASF ⁽³⁾ Tegosoft^{®} APM; origin: Evonik ⁽⁴⁾ Irgasan^{®} DP 300; origin: BASF | | |

Deodorant stick formulation is btained by weighing all the components of Part A and heating to 70-75°C. Ceteareth-25 is added once the other Part A ingredients are mixed and heated. When the Ceteareth-25 is dissolved, stearic acid is added. Part B is prepared by dissolving Triclosan in 1,2-propylene glycol. Evaporated water is compensated. Then, slowly, under mixing, Part B is poured into Part A. A perfume oil being the invention's composition of example 4 (Phase C) is added under gentle shaking. To stock, a plastic bag is put into the bucket to be sealed after cooling. Moulds were filled at about 70°C.

### Example 17

### Preparation of deodorant roll-on formulations comprising the invention's composition of matter

**Table 12: Composition of deodorant roll-on formulation**

| **Phase** | **Ingredients** | **Amount (% wt)** |
|---|---|---|
| **A** | Water | 50.00 |
| | Hydroxyethylcellulose ⁽¹⁾ | 0.70 |
| **B** | Ethanol (95%) | 40.00 |
| | 1,2-Propylene glycol | 5.00 |
| | Triclosan ⁽²⁾ | 0.30 |
| **C** | PEG-40 hydrogenated castor oil ⁽³⁾ | 3.00 |
| **D** | Invention's composition of Example 4 | 1 |

| | | |
|---|---|---|
| ⁽¹⁾ Natrosol^{®} 250 H; origin: Ashland ⁽²⁾ Irgasan^{®} DP 300; origin: BASF ⁽³⁾ Cremophor^{®} RH 40; origin: BASF | | |

Part A is prepared by sprinkling little-by-little the hydroxyethylcellulose into the water, whilst rapidly stirring with a turbine until the hydroxyethylcellulose is entirely swollen giving a limpid gel. Part B is slowly poured into Part A, whilst continuing stirring until the entire mixture is homogeneous. Then Parts C and D are added under gentle shaking.

### Example 18

### Preparation of day cream base O/W emulsions comprising the invention's composition of matter

**Table 13: Composition of day cream base O/W emulsion formulation**

| **Phase** | **Ingredients** | **Amount (% wt)** |
|---|---|---|
| A | Steareth-2 (and) PEG-8 Distearate⁽¹⁾ | 5.0 |
| | Cetyl alcohol | 0.5 |
| | Ceteth-20 (AND) glyceryl stearate (and) PEG-6 stearate (and) Steareth-20 ⁽²⁾ | 4.0 |
| | Squalan ⁽³⁾ | 1.0 |
| | Paraffin oil ⁽⁴⁾ | 2.0 |
| | Petrolatum ⁽⁵⁾ | 5.5 |
| B | Deionized water | 75.9 |
| | Propylene glycol | 5.0 |
| C | Phenoxyethanol (AND) Piroctone olamine ⁽⁶⁾ | 0.6 |
| D | Sodium carbomer ⁽⁷⁾ | 0.2 |
| E | Perfume oil | 0.3 |

| | | |
|---|---|---|
| ⁽¹⁾ Arlacel^{®} 985; origin: Croda ⁽²⁾ Tefose^{®} 2561; origin: Gattefossé ⁽³⁾ Biolip P 90; origin: Gattefossé ⁽⁴⁾ Mineral oil 30-40 CPS ⁽⁵⁾ Petroleum jelly ⁽⁶⁾ Nipaguard^{®} PO 5; origin: Clariant ⁽⁷⁾ PNC 400 | | |

Day cream base O/W emulsions is prepared by heating Phases A and B separately to 70-75 °C. Phase A is added to Phase B, then vacuum is applied. The mixture is stirred and cooled to 55°C for 15 min. After cooling to room temperature, phenoxyethanol (and) piroctone olamine (Part C) are added when a temperature of 45°C is reached. The mixture is stirred for 5 min before sodium carbomer (Part D) and a perfume oil being the invention's composition of Example 4 (Part E) is added. The mixture is stirred for 3 min, then the stirring was stopped for 15 min. When the temperature of the mixture reaches 30°C, the stirring is resumed for another 15 min until the cream becomes homogeneous, glossy and without lumps. If necessary the pH is adjusted to 6.70-7.20 with Glydant, Phenonip or Nipaguard PO5 or to 6.30-7.00 with Nikkoguard.

## Claims

1. A composition of matter comprising:
a) 0.5 to 98.8% w/w of (Z)-oxacyclohexadec-12-en-2-one;
b) 0.1 to 10% w/w of (E)-oxacyclohexadec-12-en-2-one;
c) 0 to 10% w/w of (E)-oxacyclohexadec-13-en-2-one; and
d) 0.5 to 98.8% w/w of (Z)-oxacyclohexadec-13-en-2-one.
the percentage being relative to the total weight of the composition of matter; and
wherein the weight ratio of the E-diastereoisomers to the Z-diastereoisomers is comprised in the range between 14:86 and 0.5:99.5.

2. The composition of matter according to claim 1, wherein that said composition comprises:
a) 55 to 98.8% w/w of (Z)-oxacyclohexadec-12-en-2-one;
b) 0.1 to 7% w/w of (E)-oxacyclohexadec-12-en-2-one;
c) 0 to 6% w/w of (E)-oxacyclohexadec-13-en-2-one; and
d) 0.5 to 31% w/w of (Z)-oxacyclohexadec-13-en-2-one.

3. The composition of matter according to claim 1 or 2, wherein said composition comprises:
a) 90 to 98.8% w/w of (Z)-oxacyclohexadec-12-en-2-one;
b) 0.1 to 2.5% w/w of (E)-oxacyclohexadec-12-en-2-one;
c) 0 to 1% w/w of (E)-oxacyclohexadec-13-en-2-one; and
d) 0.5 to 2.5% w/w of (Z)-oxacyclohexadec-13-en-2-one.

4. The composition of matter according to claim 1, wherein said composition comprises:
a) 0.5 to 10% w/w of (*Z*)-oxacyclohexadec-12-en-2-one;
b) 0.1 to 2.5% w/w of (*E*)-oxacyclohexadec-12-en-2-one;
c) 0 to 3% w/w of (*E*)-oxacyclohexadec-13-en-2-one; and
d) 55 to 98.8% w/w of (*Z*)-oxacyclohexadec-13-en-2-one.

5. The composition of matter according to any one of claims 1 to 4, wherein the weight ratio of the *E*-diastereoisomers to the *Z*-diastereoisomers is comprised in the range between 10:90 and 1:99.

6. The composition of matter according to any one of claims 1 to 5, wherein the weight ratio of the *E*-diastereoisomers to the *Z*-diastereoisomers is comprised in the range between 9.5:90.5 and 1:99.

7. The composition of matter according to any one of claims 1 to 6, wherein the weight ratio of the *E*-diastereoisomers to the *Z*-diastereoisomers is comprised in the range between 7.5:92.5 and 1:99.

8. The composition of matter according to any one of claims 1 to 7, wherein the weight ratio of the 12-regioisomers to the 13-regioisomers is comprised in the range between 55:45 and 99:1 or in the range between 20:80 and 1:99.

9. A method to confer, enhance, improve or modify the odor properties of a perfuming composition or of a perfumed article, which method comprises adding to said composition or perfumed article an effective amount of a composition of matter as defined in any one of claims 1 to 8.

10. Use as perfuming ingredient of a composition of matter as defined in any one of claims 1 to 8.

11. A perfuming composition comprising
i) at least a composition of matter, as defined in any one of claims 1 to 8;
ii) at least one ingredient selected from the group consisting of a perfumery carrier and a perfumery base; and
iii) optionally at least one perfumery adjuvant.

12. A perfumed consumer product comprising at least composition of matter as defined in any one of claims 1 to 8 or a perfuming composition as defined in claim 11.

13. The perfumed consumer product according to claim 12, wherein the perfumery consumer product is a perfume, a fabric care product, a body-care product, a cosmetic preparation, a skin-care product, an air care product or a home care product.

14. The perfumed consumer product according to claim 13, wherein the perfumery consumer product is a fine perfume, a splash or eau de parfum, a cologne, a shave or after-shave lotion, a liquid or solid detergent optionally in the form of a pod or tablet, a fabric softener, a liquid or solid scent booster, a dryer sheet, a fabric refresher, an ironing water, a paper, a bleach, a carpet cleaner, a curtain-care product, a shampoo, a leave-on or rinse-off hair conditioner, a coloring preparation, a color-care product, a hair shaping product, a dental care product, a disinfectant, an intimate care product, a hair spray, a skin cream or lotion, a vanishing cream, a deodorant or antiperspirant, a hair remover, a tanning or sun or after sun product, a nail product, a skin cleansing, a makeup, a perfumed soap, a shower or bath mousse, oil or gel, a foot/hand care product, a hygiene product, an air freshener, a "ready to use" powdered air freshener, a mold remover, a furnisher care, a wipe, a dish detergent or hard-surface detergent, a leather care product, a car care product.

## Patentansprüche

1. Stoffgemisch, umfassend:
a) 0,5 bis 98,8 Gew.-% (*Z*)-Oxacyclohexadec-12-en-2-on;
b) 0,1 bis 10 Gew.-% (*E*)-Oxacyclohexadec-12-en-2-on;
c) 0 bis 10 Gew.-% (*E*)-Oxacyclohexadec-13-en-2-on; und
d) 0,5 bis 98,8 Gew.-% (*Z*)-Oxacyclohexadec-13-en-2-on;
wobei sich der Prozentsatz auf das Gesamtgewicht des Stoffgemisch bezieht; und
wobei das Gewichtsverhältnis von *E*-Diastereoisomeren zu Z-Diastereoisomeren im Bereich zwischen 14:86 und 0,5:99,5 liegt.

2. Stoffgemisch nach Anspruch 1, wobei das Stoffgemisch Folgendes umfasst:
a) 55 bis 98,8 Gew.-% (*Z*)-Oxacyclohexadec-12-en-2-on;
b) 0,1 bis 7 Gew.-% (*E*)-Oxacyclohexadec-12-en-2-on;
c) 0 bis 6 Gew.-% (*E*)-Oxacyclohexadec-13-en-2-on; und
d) 0,5 bis 31 Gew.-% (*Z*)-Oxacyclohexadec-13-en-2-on;

3. Stoffgemisch nach Anspruch 1 oder 2, wobei das Stoffgemisch Folgendes umfasst:
a) 90 bis 98,8 Gew.-% (*Z*)-Oxacyclohexadec-12-en-2-on;
b) 0,1 bis 2,5 Gew.-% (*E*)-Oxacyclohexadec-12-en-2-on;
c) 0 bis 1 Gew.-% (*E*)-Oxacyclohexadec-13-en-2-on; und
d) 0,5 bis 2,5 Gew.-% (*Z*)-Oxacyclohexadec-13-en-2-on.

4. Stoffgemisch nach Anspruch 1, wobei das Stoffgemisch Folgendes umfasst:
a) 0,5 bis 10 Gew.-% (*Z*)-Oxacyclohexadec-12-en-2-on;
b) 0,1 bis 2,5 Gew.-% (*E*)-Oxacyclohexadec-12-en-2-on;
c) 0 bis 3 Gew.-% (*E*)-Oxacyclohexadec-13-en-2-on; und
d) 55 bis 98,8 Gew.-% (*Z*)-Oxacyclohexadec-13-en-2-on.

5. Stoffgemisch nach einem der Ansprüche 1 bis 4, wobei das Gewichtsverhältnis von *E*-Diastereoisomeren zu Z-Diastereoisomeren zwischen 10:90 und 1:99 liegt.

6. Stoffgemisch nach einem der Ansprüche 1 bis 5, wobei das Gewichtsverhältnis von *E*-Diastereoisomeren zu Z-Diastereoisomeren zwischen 9,5:90,5 und 1:99 liegt.

7. Stoffgemisch nach einem der Ansprüche 1 bis 6, wobei das Gewichtsverhältnis von E-Diastereoisomeren zu Z-Diastereoisomeren zwischen 7,5:92,5 und 1:99 liegt.

8. Stoffgemisch nach einem der Ansprüche 1 bis 7, wobei das Gewichtsverhältnis von 12-Regioisomeren zu 13-Regioisomeren im Bereich zwischen 55:45 und 99:1 oder im Bereich zwischen 20:80 und 1:99 liegt.

9. Verfahren zum Verleihen, Verstärken, Verbessern oder Modifizieren der Geruchseigenschaften einer Parfümierungszusammensetzung oder eines parfümierten Artikels, wobei das Verfahren die Zugabe einer wirksamen Menge eines Stoffgemisch gemäß einem der Ansprüche 1 bis 8 zu der Zusammensetzung oder dem parfümierten Artikel umfasst.

10. Verwendung eines Stoffgemischs gemäß einem der Ansprüche 1 bis 8 als Parfümierungsbestandteil.

11. Parfümierungszusammensetzung, umfassend
i) mindestens ein Stoffgemisch gemäß einem der Ansprüche 1 bis 8;
ii) mindestens einen Bestandteil, der aus der Gruppe bestehend aus einem Parfümträger und einer Parfümgrundlage ausgewählt ist; und
iii) gegebenenfalls mindestens ein Parfümadjuvans.

12. Parfümiertes Konsumprodukt, umfassend mindestens ein Stoffgemisch gemäß einem der Ansprüche 1 bis 8 oder eine Parfümierungszusammensetzung gemäß Anspruch 11.

13. Parfümiertes Konsumprodukt nach Anspruch 12, wobei es sich bei dem Parfüm-Konsumprodukt um ein Parfüm, ein Textilpflegeprodukt, ein Körperpflegeprodukt, eine Kosmetikzubereitung, ein Hautpflegeprodukt, ein Luftpflegeprodukt oder ein Haushaltspflegeprodukt handelt.

14. Parfümiertes Konsumprodukt nach Anspruch 13, wobei es sich bei dem Parfüm-Konsumprodukt um ein Feinparfüm, ein Splash oder Eau de Parfum, ein Eau de Cologne, eine Rasier- oder After-Shave-Lotion, ein flüssiges oder festes Waschmittel, gegebenenfalls in Form eines Pods oder einer Tablette, einen Textilweichmacher, einen flüssigen oder festen Duftverstärker, ein Trocknertuch, einen Textilauffrischer, ein Bügelwasser, ein Papier, ein Bleichmittel, einen Teppichreiniger, ein Vorhangpflegemittel, ein Shampoo, einen Conditioner, der im Haar verbleibt oder ausgespült wird, ein Färbepräparat, ein Farbpflegeprodukt, ein Haarformungsprodukt, ein Zahnpflegeprodukt, ein Desinfektionsmittel, ein Intimpflegeprodukt, ein Haarspray, eine Hautcreme oder -lotion, eine Abdeckcreme, ein Deodorant oder Antitranspirant, einen Haarentferner, ein Bräunungs-, Sonnen- oder After-Sun-Produkt, ein Nagelprodukt, ein Hautreinigungsmittel, ein Make-up, eine parfümierte Seife, einen Dusch- oder Badeschaum, ein Dusch- oder Badeöl oder -gel, ein Fuß-/Handpflegeprodukt, ein Hygieneprodukt, einen Lufterfrischer, einen gebrauchsfertigen pulverförmigen Lufterfrischer, einen Schimmelentferner, ein Möbelpflegemittel, ein Wischtuch, ein Geschirrspülmittel oder Reinigungsmittel für harte Oberflächen, ein Lederpflegeprodukt oder ein Autopflegeprodukt handelt.

## Revendications

1. Composition de matière comprenant :
a) 0,5 à 98,8 % p/p de (*Z*)-oxacyclohexadéc-12-én-2-one ;
b) 0,1 à 10 % p/p de (*E*)-oxacyclohexadéc-12-én-2-one ;
c) 0 à 10 % p/p de (*E*)-oxacyclohexadéc-13-én-2-one ; et
d) 0,5 à 98,8 % p/p de (*Z*)-oxacyclohexadéc-13-én-2-one. le pourcentage étant par rapport au poids total de la composition de matière ; et
dans laquelle le rapport pondéral des diastéréoisomères E sur les diastéréoisomères Z est compris dans la plage entre 14:86 et 0,5:99,5.

2. Composition de matière selon la revendication 1, dans laquelle ladite composition comprend :
a) 55 à 98,8 % p/p de (*Z*)-oxacyclohexadéc-12-én-2-one ;
b) 0,1 à 7 % p/p de (*E*)-oxacyclohexadéc-12-én-2-one ;
c) 0 à 6 % p/p de (*E*)-oxacyclohexadéc-13-én-2-one ; et
d) 0,5 à 31 % p/p de (*Z*)-oxacyclohexadéc-13-én-2-one.

3. Composition de matière selon la revendication 1 ou 2, dans laquelle ladite composition comprend :
a) 90 à 98,8 % p/p de (*Z*)-oxacyclohexadéc-12-én-2-one ;
b) 0,1 à 2,5 % p/p de (*E*)-oxacyclohexadéc-12-én-2-one ;
c) 0 à 1 % p/p de (*E*)-oxacyclohexadéc-13-én-2-one ; et
d) 0,5 à 2,5 % p/p de (*Z*)-oxacyclohexadéc-13-én-2-one.

4. Composition de matière selon la revendication 1, dans laquelle ladite composition comprend :
a) 0,5 à 10 % p/p de (*Z*)-oxacyclohexadéc-12-én-2-one ;
b) 0,1 à 2,5 % p/p de (*E*)-oxacyclohexadéc-12-én-2-one ;
c) 0 à 3 % p/p de (*E*)-oxacyclohexadéc-13-én-2-one ; et
d) 55 à 98,8 % p/p de (*Z*)-oxacyclohexadéc-13-én-2-one.

5. Composition de matière selon l'une quelconque des revendications 1 à 4, dans laquelle le rapport pondéral des diastéréoisomères *E* sur les diastéréoisomères Z est compris dans la plage entre 10:90 et 1:99.

6. Composition de matière selon l'une quelconque des revendications 1 à 5, dans laquelle le rapport pondéral des diastéréoisomères *E* sur les diastéréoisomères Z est compris dans la plage entre 9,5:90,5 et 1:99.

7. Composition de matière selon l'une quelconque des revendications 1 à 6, dans laquelle le rapport pondéral des diastéréoisomères *E* sur les diastéréoisomères Z est compris dans la plage entre 7,5:92,5 et 1:99.

8. Composition de matière selon l'une quelconque des revendications 1 à 7, dans laquelle le rapport pondéral des régioisomères 12 sur les régioisomères 13 est compris dans la plage entre 55:45 et 99:1 ou dans la plage entre 20:80 et 1:99.

9. Procédé pour conférer, augmenter, améliorer ou modifier les propriétés odorantes d'une composition parfumante ou d'un article parfumé, lequel procédé comprend l'ajout à ladite composition ou à l'article parfumé d'une quantité efficace d'une composition de matière telle que définie dans l'une quelconque des revendications 1 à 8.

10. Utilisation comme ingrédient parfumant d'une composition de matière telle que définie dans l'une quelconque des revendications 1 à 8.

11. Composition parfumante comprenant
i) au moins une composition de matière, telle que définie dans l'une quelconque des revendications 1 à 8 ;
ii) au moins un ingrédient choisi dans le groupe constitué par un support de parfumerie et une base de parfumerie ; et
iii) éventuellement au moins un adjuvant de parfumerie.

12. Produit de consommation parfumé comprenant au moins une composition de matière telle que définie dans l'une quelconque des revendications 1 à 8 ou une composition parfumante telle que définie dans la revendication 11.

13. Produit de consommation parfumé selon la revendication 12, dans lequel le produit de consommation parfumé est un parfum, un produit d'entretien de tissu, un produit de soin du corps, une préparation cosmétique, un produit de soin de la peau, un produit d'assainissement de l'air ou un produit d'entretien ménager.

14. Produit de consommation parfumé selon la revendication 13, dans lequel le produit de consommation parfumé est un parfum fin, une éclaboussure ou une eau de parfum, une eau de Cologne, une lotion de rasage ou d'après-rasage, un détergent liquide ou solide éventuellement sous forme de dosette ou de comprimé, un assouplissant, un rehausseur de parfum liquide ou solide, une feuille de séchage, un désodorisant pour tissus, une eau de repassage, un papier, un agent de blanchiment, un nettoyant pour tapis, un produit d'entretien des rideaux, un shampoing, un après-shampoing sans rinçage ou à rincer, une préparation colorante, un produit d'entretien des couleurs, un produit de mise en forme des cheveux, un produit de soin dentaire, un désinfectant, un produit de soin intime, une laque pour cheveux, une crème ou une lotion pour la peau, une crème de jour, un déodorant ou un antitranspirant, un épilateur, un produit de bronzage ou de soleil ou d'après-soleil, un produit pour les ongles, un nettoyant pour la peau, un maquillage, un savon parfumé, une mousse, une huile ou un gel pour la douche ou le bain, un produit de soin des pieds/mains, un produit d'hygiène, un nm assainisseur d'air, un assainisseur d'air en poudre « prêt à l'emploi », un anti-moisissure, un produit d'entretien des meubles, une lingette, un détergent à vaisselle ou pour surfaces dures, un produit d'entretien du cuir, un produit d'entretien de la voiture.
